# EUROPEAN PATENT SPECIFICATION

(11) **EP 3 789 499 B1**
(45) Date of publication and mention of the grant of the patent: **15.03.2023**
(21) Application number: 20197453.2
(22) Date of filing: 13.02.2013
(51) Int. Cl.: C12Q 1/68

(54) **BLOOD COLLECTION DEVICE FOR IMPROVED NUCLEIC ACID REGULATION**
BLUTSAMMELVORRICHTUNG ZUR STABILISIERUNG ZELLFREIER PLASMA-DNA
DISPOSITIF DE PRÉLÈVEMENT SANGUIN PERMETTANT D'AMÉLIORER LA RÉGULATION D'ACIDE NUCLÉIQUE

(30) Priority: 13.02.2012 US 201261597908 P; 14.07.2012 US 201261671681 P
(43) Date of publication of application: 10.03.2021
(62) Divisional of application: 17170896.9
(73) Proprietor: Streck, Inc., La Vista, NE 68182 (US)
(72) Inventor: RYAN, Wayne L., deceased (US); FERNANDO, Rohan M., Omaha, Nebraska 68136 (US); DAS, Kausik, Lincoln, Nebraska 68510 (US)
(74) Representative: Kutzenberger Wolff & Partner

(56) References cited:
- WO-A1-2010/123908
- US-A1- 2010 184 069
- Schatz et al.: "PRESERVATION OF CELL-FREE DNA IN STORED BLOOD SAMPLES FOR THE ANALYSIS OF THE (M)SEPT9 COLORECTAL CANCER SCREENING MARKER ENABLES SAMPLE SHIPMENT BY MAIL", , May 2011 (2011-05), XP002699458, Published as a poster at the conference on international federation of clinical chemistry and laboratory medicine Worldlab and EU, Berlin, Germany Retrieved from the Internet: URL:http://www.epiprocolon.com/uploads/tx_ txpublicationslist/epi-poster-IFCC-1-A4.pd f [retrieved on 2013-06-25]

## Description

### FIELD OF THE INVENTION

The present application is a divisional application of European patent application no. 17 170 896.9 / 3 225 699, which in turn was a divisional application of European patent application no. 13 706 856.5 / 2 814 981, which in turn matured as European regional stage application from international application no. PCT/US2013/025912 / WO 2013/123030.

The teachings herein relate to devices and methods for stabilizing and preserving cell-free DNA without damaging DNA integrity for improved protection and regulation of nucleic acid materials during collection, storage, and shipment. These devices are not claimed as such. In the following, references to these devices essentially serve information purposes.

### BACKGROUND OF THE INVENTION.

Cell-free DNA (cfDNA) naturally occurs in blood and has been largely attributed to apoptotic and necrotic processes. While the presence of cfDNA in blood was discovered in 1948, its implications in clinical medicine were not realized for more than two decades. Specifically, it was demonstrated that cfDNA was present in the serum of patients with systemic lupus erythematosus and that cfDNA levels were elevated in the serum of cancer patients. These findings sparked an interest in the potential use of cfDNA in disease diagnosis and prognosis. Investigations carried out with rheumatoid arthritis, colorectal, breast, pancreatic, head and neck cancer patients have all shown a marked rise in cfDNA concentrations. The cfDNA extracted from plasma or serum of cancer patients has shown characteristics typical of tumor DNA and may serve as non-invasive biomarkers for cancer detection and management.

There is also growing interest in the potential use of cell-free fetal nucleic acids in non-invasive prenatal diagnosis. Lo et al. Lancet 350 (1997) 485-487 were the first to show that pregnant donor blood samples have elevated maternal cfDNA concentrations and also demonstrated the presence of fetal cfDNA in maternal plasma. Clinical applications involving fetal cfDNA analysis include sex determination, single-gene disorders, pregnancy-related disorders and aneuploidy detection.

Since that time, a cumulative body of research has identified cfDNA as both a prognostic and diagnostic indicator for multiple pathogenic conditions; i.e., cancer-associated genetic and epigenetic alterations, fetal DNA mutation and prenatal diagnosis, and viral infection-through viral DNA detection in human blood. As such, accurate detection of cfDNA in human biological specimens is becoming a mainstream, non-invasive avenue that allows assessment, screening, and disease classification and monitoring during routine clinical analysis.

cfDNA refers to DNA fragments detectable in multiple body fluids. Plasma or serum are most frequently used for this purpose, however, the presence of cfDNA has been detected in urine, saliva, feces, synovial fluid, cerebrospinal fluid, and peritoneal fluid. The average circulating concentration of cfDNA for a healthy individual is 30 ng/ml, the cfDNA is generally double stranded, and approximately 0.18-21 kilobases in size (Wagner, J. "Free DNA--new potential analyte in clinical laboratory diagnostics?" Biochem Med (Zαgreb) 22(1): 24-38). The detection of cfDNA, however, is particularly challenging for the following reasons: (1) during prenatal diagnosis, the predominant maternal cellular materials can interfere with fetal cfDNA detection, (2) sample processing after collection can induce cell lysis, leading to aberrant increases in the amount of circulating cfDNA, and (3) the relatively low level of cfDNA underscores the potential risks of generating false negative results due to the loss of scarce target cfDNA sequences-due to sample instability or inappropriate sample processing. To this end, strategies to minimize contaminating cellular DNA and preserve cfDNA have included various pre-analytical factors such as the type of blood collection tubes, sample storage conditions, and centrifugation protocols. For example, anticoagulants such as EDTA, heparin, and citrate prevent clotting of whole blood cells, which is thought to reduce DNA release from the leukocyte cell population. Also, the optimization of centrifugation conditions is required to prevent lysis but adequately separate intact cells from cell-free plasma.

Due to the low abundance of the cfDNA biomarkers, it is recommended that genomic DNA (gDNA) background levels be minimized to provide accurate measurements cfDNA levels. It is further beneficial that the structural integrity of the cfDNA be maintained due to the minimal amounts available for analysis. It is therefore necessary to address several pre-analytical issues that arise during the time between blood draw and subsequent DNA isolation. These issues include delays in blood processing, blood storage temperature, and agitation of the sample during transport and shipment of blood. Such conditions may alter plasma DNA (pDNA) levels by causing gDNA release from lysed nucleated blood cells and obfuscate true cfDNA. As a result, it is important to consider the type of blood collection device and post-phlebotomy conditions while working with cfDNA samples.

Previous efforts have focused on chemical methods, such as the use of formaldehyde-based fixation to enrich the fractional concentration of cfDNA and extend the time post-venipuncture that a sample can be effectively analyzed. However, studies examining the effectiveness of formaldehyde preservation of whole blood for extended-term analysis of cfDNA concentrations in biological samples have provided statistical inconsistencies. For example, it has been demonstrated that treating a blood sample with formaldehyde immediately after blood draw had no beneficial effects on the preservation of the proportion of fetal cfDNA in maternal plasma. (Chinnapapagari SKR, Holzgreve W, Lapaire O, et al. 2004. Treatment of maternal blood samples with formaldehyde does not alter the proportion of circulatory fetal nucleic acids (DNA and RNA) in maternal plasma. Clin Chem 51:652-655 and Chiu RWK, Chan KCA, Lau TK, et al. 2004. Lack of dramatic enrichment of fetal DNA in maternal plasma by formaldehyde treatment. Clin Chem 51:655-658). It has also been reported that formaldehyde has detrimental effects on plasma nucleic acids (Chung GTY, Chiu RWK, Chan KCA, Lau TK, Leung TN, Chan LW and LO YMD. 2005. Detrimental effect of formaldehyde on plasma RNA detection. Clin Chem 51:1074-1076). This suggested that, although formaldehyde fixation may prevent contamination of cfDNA concentrations by cellular DNA, formaldehyde and formaldehyde-based chemical stabilizers alone may be inadequate for both the accurate and precise analysis of cfDNA concentration over extended time periods prior to sample analysis. WO 2010/123908 A1 discloses compositions for collecting, storing and transporting populations of nucleic acids from biological specimens, and clinical, forensic, or environmental samples.

There is thus a need for methods of stabilizing and protecting cfDNA whereby structural integrity is maintained and genomic background DNA is minimized, so that shipping and storage is possible with minimal effect on the cfDNA. There is a further need for such methods where the detrimental effects of aldehyde fixation are avoided.

### SUMMARY OF THE INVENTION

The teachings herein employ a protocol using a unique protective agent composition that successfully preserves samples while stabilizing DNA integrity for a prolonged period (e.g., which may be at least 14 days, and which may be at room temperature) using several analytical techniques. The present teachings provide a consistent and efficient method for preserving nucleic acids in biological samples, and particularly preserving DNA in plasma. Data demonstrated herein describes a method that reduces blood cell lysis, nuclease activity, and permits accurate and precise analytical analysis by virtue of the preservation of the final concentration of recoverable cfDNA over time. In so doing, the teachings provide a novel approach that improves the downstream clinical analysis of cfDNA in plasma. The present invention prevents contamination of plasma cfDNA with cellular DNA (e.g., genomic DNA or gDNA) that is released from damaged cells by stabilizing blood cells within a sample. The present teachings describe protecting the cfDNA by inhibiting deoxyribonuclease activity in plasma. As a result of nucleated blood cell stabilization, it is no longer necessary to separate plasma immediately after venipuncture. Furthermore, samples can be stored at room temperature for up to 14 days without deleterious effects to sample integrity, which eliminates the need for cold storage of the plasma sample.

The present teachings further provide for blood collection devices that reduce background levels of genomic DNA (gDNA) in plasma compared to K₃EDTA tubes, when subjected to conditions that may occur during sample storage and shipping. In one aspect, the present teachings contemplate a method for blood sample treatment comprising locating a protective agent into the blood collection devices described herein. The protective agent may include a preservative. A blood sample may be drawn into the blood collection device, the blood sample having a first pDNA concentration. The blood collection device containing a blood sample may be transported from a first location to a second location, wherein at least a portion of the transporting occurs at a temperature of greater than about 0°C. The cfDNA from the sample may be isolated at least 24 hours after blood draw, the sample having a second pDNA concentration, wherein the second pDNA concentration is not higher than the first pDNA concentration by any statistically significant value. This method for blood sample treatment is not claimed. Further, these blood collection devices are not claimed as such. In the following, references to this method for blood sample treatment and references to these blood collection devices essentially serve information purposes.

The teachings herein further include that the preservative may be selected from the group consisting of diazolidinyl urea and imidazolidinyl urea. The concentration of the preservative prior to the contacting step may be between about 0.1 g/ml and about 3 g/ml. The cell-free DNA may be isolated from the sample at least 3 days after blood draw. The cell-free DNA may be isolated from the sample at least 7 days after blood draw. The cell-free DNA may be isolated from the sample at least 14 days after blood draw. The sample may have a first gDNA concentration at blood draw and a second gDNA concentration after transporting and the second gDNA concentration is not higher than the first gDNA concentration by any statistically significant value. The transporting step may occur without freezing the blood sample to a temperature colder than about -30°C. The protective agent may contact the cell-free DNA so that after a period of at least 7 days from the time the blood sample is drawn, the amount of cell-free DNA is at least about 90% of the amount of cell-free DNA at the time the blood sample is drawn. The protective agent may contact the cell-free DNA so that after a period of at least 7 days from the time the blood sample is drawn, the amount of cell-free DNA present in the sample is about 100% of the amount of cell-free DNA present in the sample at the time the blood sample is drawn. These preferred embodiments relate to the method for blood sample treatment which is not claimed.

The teachings herein contemplate improved protective agent compositions, and methods of stabilizing a biological sample for analysis.

The invention relates to a method of stabilizing a biological sample for analysis, comprising the steps of:
a. obtaining in a sample collection container a biological sample from a subject selected from a victim of a crime, a suspect of a crime, a subject undergoing detection and/or monitoring of a cancerous condition, a subject undergoing detection and/or monitoring of a neurogenerative condition, a subject undergoing detection and/or monitoring of a psychiatric condition, or a subject who is not pregnant, wherein the biological sample includes at least one circulating cell-free first nucleic acid from the subject;
b. contacting the biological sample while within the sample collection container with a protective agent composition that includes
   - at least one preservative agent selected from diazolidinyl urea, imidazolidinyl urea, dimethoylol-5,5-dimethylhydantoin, dimethylol urea, 2-bromo-2.-nitropropane-1,3-diol, oxazolidines, sodium hydroxymethyl glycinate, 5-hydroxymethoxymethyl- 1 -1 aza-3,7 -dioxabicyclo- [3.3.0] octane, 5-hydroxymethyl-1-1aza-3,7dioxabicyclo[3.3.0]octane, 5-hydroxypoly-[methyleneoxy]methyl-1-1aza-3,7dioxabicyclo[3.3.0]octane, quaternary adamantine or any combination thereof,
   - an optional anticoagulant, and
   - a quenching agent to form a mixture that includes the protective agent composition and the sample, wherein the contacting step includes employing as the quenching agent an ingredient selected from glycine, lysine, ethylene diamine, arginine, urea, adenine, guanine, cytosine, thymine, spermidine, or any combination thereof;
c. quenching any free formaldehyde that may be present with the quenching agent from the protective agent composition so that the free formaldehyde reacts to form a reaction product that is inert to the first nucleic acid of the biological sample, the resulting mixture is devoid of any aldehyde, and nucleic acids within the sample are suitable for polymerase chain reaction and DNA sequencing, and DNA and are substantially devoid of aldehyde induced (i) nucleic acid (e.g., DNA) to protein cross-linking, (ii) nucleic acid (e.g., DNA) to nucleic acid (e.g., DNA) intra-molecular and/or inter-molecular cross-links; or both (i) and (ii), to thereby form a sample that is amplifiable by polymerase chain reaction (PCR), and DNA sequencing;
wherein the biological sample comes from or is in the initial form of one or more biological matter selected from urine, saliva, feces, synovial fluid, cerebrospinal fluid, or peritoneal fluid.

For samples derived from blood, there may be a blood draw step of drawing blood from a patient into a blood collection device that has the protective agent composition loaded therein prior to the blood draw step. According to the invention, the biological sample comes from or is in the initial form of one or more biological matter selected from urine, saliva, feces, synovial fluid, cerebrospinal fluid, or peritoneal fluid. Blood is not covered by the Markush-Group defining biological matter according to the invention. In the following, references to blood as being biological matter essentially serve information purposes. The method may include a step of transporting the sample while it is contacted with the protective agent composition from a blood draw site to a clinical laboratory (e.g., one located at least 100 meters, 1000 meters, 10,000 meters from the blood draw site) at which a sample analysis will occur. The quenching occurs prior to and/or substantially simultaneously with the contacting step. The methods may include a step of isolating cell-free DNA from the sample. The methods may be free of any step of centrifugation of the sample. The methods may be free of any step of isolating cell-free fetal DNA from maternal blood. The methods may be free of any step of refrigerating the sample (e.g., to a temperature below room temperature, such as about 10°C or cooler) after it has been contacted with the protective agent composition.

As can be appreciated from the above, the teachings herein provide for advantageous treatment of DNA-containing samples and provide stabilized samples that are essentially free of detectable covalent modifications that inhibit PCR amplification, such as by inhibiting polymerase binding and elongation, primer annealing, and/or DNA dye intercalation (such as with SYBR green or ethidium bromide). Any modifications to nucleic acids (e.g., DNA) that may inhibit accurate PCR analysis and DNA sequencing as a result of treatment with the protective agent compositions of the present teachings is delayed for at least 24, 48, or 96 hours, 1 week or even two weeks. There is a substantial absence of any indication of protective agent composition interaction with DNA that is random in nature, increases over time, or both. Thus, use of the teachings herein enables greater predictability in helping to assure that any DNA sequence of interest that is to be amplified or sequenced will not be damaged. This provides an advantage over treating DNA with just formaldehyde. That is, it is believed that formaldehyde modification will immediately prevent amplification of some genes and the number of genes that are not amplified will increase with time. There is no predicting which genes will be modified first. As a result, a clinician may be unable to detect certain biomarkers, and/or other gene characteristics (such as critical gene mutations or deletions that effect fetal development, in the context of cell-free fetal DNA analysis).

### BRIEF DECSRIPTION OF THE DRAWINGS

Fig. 1a shows a graph displaying the effect of an exemplary protective agent composition on pDNA detection by qPCR at 3 and 6 hours post blood draw.
Fig. 1b shows a graphic representation of the effect of an exemplary protective agent composition in accordance with the teachings herein on pDNA detection by qPCR at 7 and 14 days post blood draw.
Fig. 2a shows a graphic representation of the effect of elevated background gDNA in plasma on the detection of rare DNA sequences.
Fig. 2b shows a graphic representation of the effect of elevated background gDNA in plasma on the detection of rare DNA sequences and the effect of an exemplary protective agent composition in accordance with the teachings herein.
Figs. 3a and 3b show graphic representations of the effect of shaking and shipping on pDNA concentration in blood samples, including blood samples treated with an exemplary protective agent composition in a blood collection device in accordance with the present teachings and blood samples in standard K₃EDTA tubes.
Figs. 4a and 4b show graphic representations of the effect of storage temperature on pDNA concentration in blood samples, including blood samples treated with an exemplary protective agent composition in a blood collection device in accordance with the present teachings and blood samples in standard K₃EDTA tubes.
Fig. 5 is a schematic depiction to illustrate potential interactions between an aldehyde (e.g., formaldehyde) and a genomic DNA sample.
Figs. 6a and 6b depict the schematic diagrams of one of the several modes of DNA-DNA and one of the several modes of DNA-protein cross-link reactions.
Fig. 7a is a ¹³C nuclear magnetic resonance (NMR) read-out for a sample treated with an exemplary protective agent composition in accordance with the present teachings, illustrating the substantial absence of any peak at 82 ppm, which supports that the treated sample is essentially free of any free formaldehyde.
Fig. 7b is a comparative ¹³C (NMR) read-out for a formaldehyde sample in accordance with the present teachings, illustrating the characteristic presence of a peak at 82 ppm.

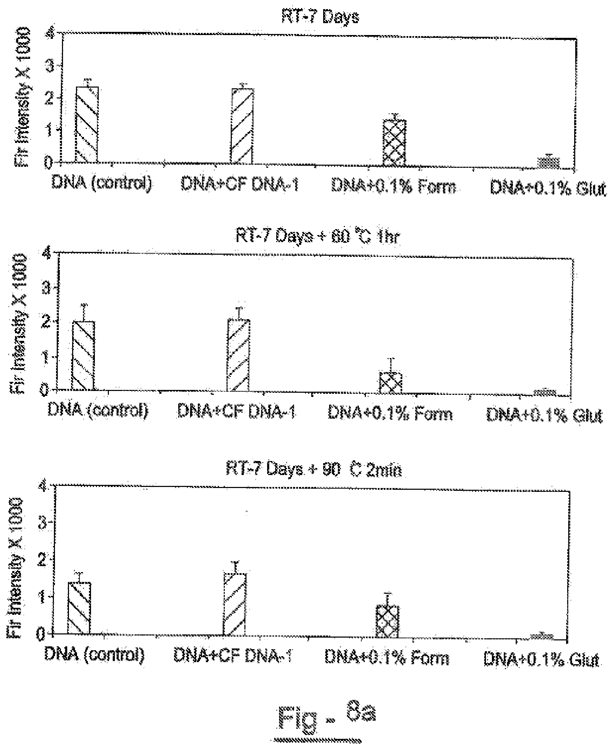

Fig. 8a is a series of fluorescence (flr) spectrometry intensity plots to compare a DNA containing control sample ("DNA (control)") and DNA-containing samples treated with one of a protective agent composition ("DNA+CF DNA-1") in accordance with the present teachings, formaldehyde ("DNA+0.1% Form") or glutaraldehyde ("DNA+0.1% Glut"); with the series showing samples after seven days at room temperature (Upper plot: "RT 7 Days"), samples after seven days at room temperature followed by heating for one hour at 60°C (Middle plot: "RT 7 Days + 60°C- 1hr"), and samples after seven days at room temperature followed by heating for two minutes at 90°C (Middle plot: "RT 7 Days + 90°C- 2 min").

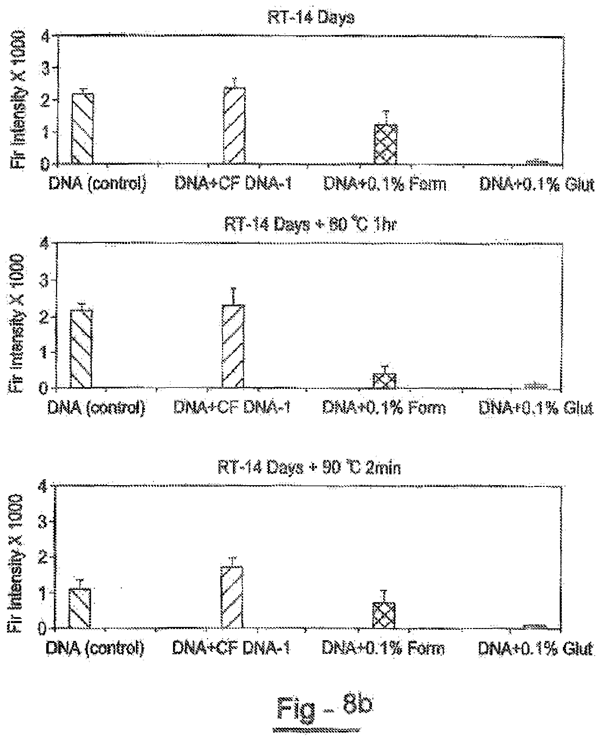

Fig. 8b is a series of fluorescence (flr) spectrometry intensity plots to compare a DNA containing control sample ("DNA (control)") and DNA-containing samples treated with one of a protective agent composition ("DNA+CF DNA-1") in accordance with the present teachings, formaldehyde ("DNA+0.1% Form") or glutaraldehyde ("DNA+0.1% Glut"); with the series showing samples after fourteen days at room temperature (Upper plot: "RT 14 Days"), samples after fourteen days at room temperature followed by heating for one hour at 60°C (Middle plot: "RT-14 Days + 60°C- 1hr"), and samples after fourteen days at room temperature followed by heating for two minutes at 90°C (Middle plot: "RT-14 Days + 90°C- 2 min")
Figs. 9a-9c are gel electrophoresis images that illustrate and compare the PCR amplification of untreated DNA (control DNA); DNA samples treated with a composition in accordance with the present teaching (denoted by DNA+CF-DNA-BCT), DNA samples treated with formaldehyde (denoted by DNA+Form) and DNA samples treated with glutaraldehyde (denoted by DNA+Glut).
Figs. 10a-10d are plots indicating real time quantitative analysis results for polymerase chain reaction (PCR) amplification of DNA samples treated with one of a protective agent composition ("DNA+cfDNA-BCT") in accordance with the present teachings in which a sample is drawn into a blood collection device containing a protective agent composition of the present teachings, formaldehyde ("DNA+0.1% form") or glutaraldehyde ("DNA+0.1% glut), and compared with an untreated DNA control sample ("DNA Control").
Fig. 11 shows a graphic representation indicating real time quantitative analysis for polymerase chain reaction (PCR) amplification of DNA-containing samples treated with one of a protective agent composition ("DNA+cfDNA-BCT") in accordance with the present teachings in which a sample is drawn into a blood collection device containing a protective agent composition of the present teachings, formaldehyde ("DNA+0.1% formaldehyde") or glutaraldehyde ("DNA+0.1% glutaraldehyde), and compared with an untreated DNA control sample ("DNA Control").
Figs. 12a and 12b are plots of circular dichroism spectra illustrating plots of native-DNA control (the curve denoted "DNA-RT" that starts bottom-most at the intersection with the y-axis); DNA sample treated in accordance with the present teachings (the curve denoted "DNA+CF DNA BCT-RT" that starts at the highest location at its intersection with the y-axis); and DNA sample treated with formaldehyde (the curve denoted "DNA=0.1%form RT").
Figs. 13a-13d are agarose gel electrophoresis images that illustrate a number of parallel lanes and compare untreated control samples (denoted by "Native DNA"; lanes 1, 2, 8 and 9); DNA samples treated with a protective agent composition in accordance with the present teachings (denoted by "DNA+cfDNA"; lanes 3, 4, 10 and 11); DNA samples treated with only imidazolidinyl urea (denoted by "DNA+IDU"; lanes 5 and 12); samples treated with formaldehyde (denoted by "DNA+0.1% form"; lanes 6 and 13); and samples treated with glutaraldehyde (denoted by "DNA+0.1% glut"; lanes 7 and 14).
Fig. 14 shows a graphic representation of the effect of DNase treatment of plasma on cfDNA concentration.
Fig. 15 shows a graphic representation of the effect of storage on cfDNA concentration in blood samples in K₃EDTA tubes.
Fig. 16 shows a graphic representation of the effect of storage on cfDNA concentration in blood samples in blood collection devices in accordance with the present teachings.
Fig. 17 shows the chemical equation for an exemplary quenching step in accordance with the present teachings.
Fig. 18 shows the chemical equation for an additional exemplary quenching step in accordance with the present teachings.
Fig. 19 shows a graphic representation of formaldehyde release and quenching with various agents.
Fig. 20 shows hydrated formaldehyde presence with various quenching agents.
Fig. 21 shows formaldehyde concentrations in the presence of various quenching agents.
Fig. 22 shows possible chemical equations for the release of hydrated formaldehyde.
Fig. 23 shows chemical presence in glycine/formaldehyde mixture.
Fig. 24 shows the chemical equation for the reaction of glycine and formaldehyde at room temperature.
Fig. 25 shows the chemical equation for the reaction of glycine and formaldehyde at 50°C for 4 days.
Fig. 26 shows possible chemical equations for formaldehyde release and subsequent formaldehyde/glycine reaction.
Fig. 27 shows the chemical equation for quencher agent/formaldehyde reaction.

### DETAILED DESCRIPTION

Unless otherwise stated, percentages as set forth herein refer to percent by weight. Further, unless the context of the discussion makes clear to the contrary, references to nucleic acid may include ribonucleic acid (RNA), deoxyribonucleic acid (DNA), or fragments of either. Thus, for example, discussion of cell-free DNA (cfDNA) refers not only to cell-free DNA, but to DNA fragments.

The present teachings contemplate a non-invasive genetic screening method for the identification of DNA sequence characteristics that are potentially indicative of various pathological conditions, including but not limited to: cancer, a neurogenerative disorder, a psychiatric disorder, viral infection, prenatal diagnosis, autoimmune diseases, microchimerism, acute myocardial infarction, stroke, mesenteric ischemia, and pancreatitis. The teachings herein envision not only preserving the state of any cells in a sample (e.g., by stabilizing the cell membranes so that nucleic acids are prevented from being released from within the cells), but also envisions protecting the cell-free DNA from adverse effects of deoxyribonuclease activity. The methods of the teachings herein generally involve steps of contacting a biological sample (which sample optionally may be free of any fetal DNA (e.g., it may be free of any cell-free fetal DNA)), and which may include multiple blood cells and cell-free biomolecules), with an aldehyde-free (e.g., formaldehyde-free) protective agent composition in an amount and time that is sufficient to prevent (1) the release of genomic DNA into the blood sample and (2) deoxyribonuclease activity that degrades cell-free nucleic acids (e.g., cfDNA) in the sample. The treatment is such that it substantially prevents any free aldehyde from adversely reacting with the cell-free nucleic acids of the sample (e.g., cfDNA) such as by employing a quenching agent. In this manner, substantial quantities of cfDNA can be isolated from the sample with little concern that the isolated cfDNA contains DNA released by a cell from the sample after the sample has been obtained. The nucleic acid integrity (e.g., the cfDNA integrity) is substantially preserved in its as provided state (e.g., the state at the time of blood draw) by avoiding the damaging effects of any aldehyde (e.g., formaldehyde). Thus, accurate and precise analytical analysis of the nucleic acid (e.g., cfDNA in plasma) of the sample can be achieved. The method may further include steps of analyzing the nucleic acid (e.g., cfDNA) from a sample that has been treated in accordance with the above, or that have otherwise been contacted with the protective agent composition and the quenching agent therein. As noted, the teachings herein permit identification of cfDNA characteristics for prognostic and diagnostic use of various pathological conditions in the clinic.

Aldehyde-based chemicals, such as formaldehyde and glutaraldehyde, react with a variety of nucleophilic cellular constituents, forming, for example, methylol derivatives of the mercaptan group of glutathione and the amino groups of RNA, DNA, and protein. In addition, these aldehydes act as cross-linking agents that produce DNA-protein and DNA-DNA intra- and inter-molecular cross-links. The combination of the formaldehyde-induced DNA modifications listed above can affect DNA melting, DNA amplifiability during polymerase chain reaction (PCR) analysis and DNA sequencing. For genetic studies designed around the PCR, this can cause a decrease in cfDNA concentrations and hinder the detection of rare DNA targets after prolonged fixation with formaldehyde. In addition to the detrimental effects aldehyde-based fixatives have on PCR-based analysis of cfDNA, Ganguly *et al.* showed heterogeneities in fluorescence emission spectra on formaldehyde-fixed DNA samples, which manifested in lower overall fluorescent signal and spurious data (Ganguly S, Clayton AHA, and Chattopadhyay A. 2011 Bioche. Biophy. Res. Comm. 405: 234-237). Fixation alters fluorescence lifetime and anisotropy of cells expressing EYFP-tagged serotonin_{1A} receptor. Overall, the body of work in this field suggests that formaldehyde can successfully function as a cell preservative but is detrimental for downstream analytical analyses to measure accurate cfDNA concentrations, detect rare DNA targets and DNA sequencing, likely resulting from the adverse chemical modifications consistent with prolonged fixation of biomolecules (i.e.; DNA, RNA, and protein). Therefore, to improve qualitative and quantitative analytical analysis of cfDNA concentrations in human biological fluid, there is a clear demand for a time and cost-effective method that is formaldehyde-free and preserves the biological sample while maintaining the sample integrity, quantity, and specificity of the cfDNA to be analyzed.

The teachings herein envision that a single protective agent composition is employed that includes a preservative composition and a quenching agent. Such protective agent composition may be preloaded into a sample collection device, such as a blood collection tube (which may be evacuated to a pressure below atmospheric pressure after loading). Thus, it is possible that a sample may be taken from a subject directly into the sample collection device (e.g., a blood collection tube), at which time it will be contacted with the protective agent composition. It is also possible that a sample can be taken from a subject into a first container and the sample subsequently transferred to one or more second container in which the protective agent composition is present.

According to the invention, the aldehyde-free (e.g., formaldehyde-free) protective agent composition includes at least one preservative agent selected from the group consisting of: diazolidinyl urea, imidazolidinyl urea, dimethoylol-5,5-dimethylhydantoin, dimethylol urea, 2-bromo-2-nitropropane-1,3-diol, oxazolidines, sodium hydroxymethyl glycinate, 5-hydroxymethoxymethyl-1-laza-3,7-dioxabicyclo [3.3.0] octane, 5-hydroxymethyl-1-laza-3,7-dioxabicyclo [3.3.0] octane, 5-hydroxypoly[methyleneoxy]methyl-1-laza-3,7-dioxabicyclo [3.3.0] octane, quaternary adamantine and any combination thereof. Though the aldehyde-free (e.g., formaldehyde-free) protective agent composition may release an aldehyde (e.g., formaldehyde), the teachings herein envision a specific step of quenching the aldehyde to render it inert to the cfDNA.

The preservative composition is used in combination with a quenching agent for helping to assure that nucleic acid (e.g., DNA) in the sample avoids being subjected to free aldehyde (e.g., free formaldehyde) which may cause one or more deleterious effects upon the nucleic acid). Accordingly, the teachings herein contemplate a use of at least one aldehyde quenching agent, which is employed in an amount and in a manner sufficient so that any free aldehyde (e.g., formaldehyde) released from the protective agent composition reacts to form a reaction product that is inert to the nucleic acid of the biological sample, the resulting mixture is devoid of any aldehyde, and nucleic acids within the sample are suitable for polymerase chain reaction and DNA sequencing, and will exhibit structural integrity comparable to native nucleic acids (e.g., DNA will exhibit ellipticity that is substantially similar that of untreated native DNA, as measured by circular dichroism spectroscopy or will exhibit DNA-dye fluorescence that is substantially similar to that of untreated native DNA, as measured by fluorescence spectroscopy).

The concentration of the preservative composition prior to sample contact may be at a concentration at which cross-linking of DNA to DNA and DNA to proteins is not observed, as indicated by agarose gel electrophoresis. The concentration of the preservative composition after sample contact may be greater than about 20 mg/ml, 10 mg/ml, 5 mg/ml, 2 mg/ml or even less than about 0.8 g/ml of the mixture of protective agent composition and biological (e.g., blood) sample. The concentration of the preservative composition after sample contact may be more than about 0.1 g/ml of the mixture of protective agent composition and biological (e.g., blood) sample. By way of example, the concentration of the preservative composition after sample contact may be between approximately 0.1 g/ml to approximately 0.8 g/ml of the mixture of protective agent composition and biological (e.g., blood) sample. The concentration of the preservative composition after sample contact may be between approximately 0.3 g/ml to approximately 0.6 g/ml of the mixture of protective agent composition and biological (e.g., blood) sample. The concentration of the preservative composition both before and after contact with a blood sample may be modified depending upon what diagnostic procedures a sample may undergo. As an example, the concentration may be modified in the event that a sample is to undergo flow cytometry analysis. More specifically, the concentration may be increased in the event that a sample is to undergo flow cytometry analysis. Thus, the concentration of the preservative composition after sample contact may be greater than about 15 mg/ml, greater than about 25 mg/ml, or even greater than about 30 mg/ml after sample contact. The formulation of the protective agent composition (and the preservative composition contained therein) may also be modified such that a sample that will undergo flow cytometry analysis may contain diazolidinyl urea. The protective agent composition may also include a quenching agent. The protective agent composition may also include EDTA.

The quenching agent may be one or more compounds that include at least one functional group capable of reacting with an electron deficient functional group of an aldehyde (e.g., an amine compound that reacts with formaldehyde to form methylol and/or imine Schiff base or a cis-diol compound that reacts with formaldehyde to form a cyclic acetal). The quenching agent may be selected from amino acids, alkyl amines, polyamines, primary amines, secondary amines, ammonium salts, nucleobases or any combination thereof. According to the invention, the quenching agent is selected from glycine, lysine, ethylene diamine, arginine, urea, adinine, guanine, cytosine, thymine, spermidine, or any combination thereof.

The concentration of the quenching agent is an amount that is sufficiently large that after contacting the sample with the protective agent composition, there is an absence of free aldehyde (e.g., an absence of free formaldehyde). However, the concentration is sufficiently small that dilution of the sample will not materially impact any analyzed characteristic of the sample. The concentration of the formaldehyde-quenching reagent after the sample contacting step may be above about 0.001 g/ml, 0.002 g/ml or even about 0.004 g/ml of the mixture of protective agent composition and biological (e.g., blood) sample. The concentration of the formaldehyde-quenching reagent after the sample contacting step may be below about 0.03 g/ml, 0.01 g/ml, or even about 0.008 g/ml of the mixture of protective agent composition and biological (e.g., blood) sample. By way of example, the concentration of the formaldehyde-quenching reagent after the sample contacting step may be between about 0.004 g/ml to about 0.008 g/ml.

The teachings herein also envision the use of a quenching agent that is a combination of glycine with one or more additional quenching agents, or a quenching agent of a type and in an amount as described herein that is other than glycine. For example, such a quenching agent may be employed (as taught herein) in combination with any anticoagulant and with the preservative composition as described for treating any sample that may contain cell-free DNA (e.g., fetal cell-free DNA).

Upon being brought into contact with a sample to form a mixture of the sample and protective agent composition (e.g., at time of a blood draw into a blood collection device containing a protective agent composition of the teachings herein), the protective agent composition may be present in an overall small fraction of the mixture volume. For example, it may be present in an amount that is less than about 5%, 2%, 0.5% or even less than about 0.3% of the overall mixture volume. For example, the protective agent composition may be present in an amount of from about 1:20 parts by volume to about 1:300 parts by volume of the mixture. The amount of the protective agent composition may be present from about 1:50 parts by volume to about 1:200 parts by volume of the mixture.

During at least the contacting step, the amount of the protective agent composition is present from about 1:20 (1 part protective agent composition to 20 parts total mixture) parts by volume to about 1:300 parts by volume of the total mixture (which includes both the protective agent composition and the biological sample). For instance, during at least the contacting step, the amount of the protective agent composition is present from about 1: 100 parts by volume to about 1:200 parts by volume of the mixture.

According to the invention, the protective agent composition includes at least one preservative agent selected from diazolidinyl urea, imidazolidinyl urea, dimethoylol-5,5-dimethylhydantoin, dimethylol urea, 2-bromo-2.-nitropropane-1,3-diol, oxazolidines, sodium hydroxymethyl glycinate, 5-hydroxymethoxymethyl-1-laza-3,7-dioxabicyclo[3.3.0]octane, 5-hydroxymethyl-1 -1 aza-3,7dioxabicyclo [3.3.0]octane, 5 -hydroxypoly [methyleneoxy]methyl-1-1aza-3,7dioxabicyc-lo[3.3.0]octane, quaternary adamantine or any combination thereof. By way of illustration, the contacting step may include employing as the protective agent composition, a composition that includes imidazolidinyl urea in an amount of about 0.1 to about 2.0% by weight of the total mixture of the protective agent composition plus a biological sample; optionally, ethylenediaminetetraacetic acid (EDTA) in an amount of about 0.05 to about 0.75% by weight of the total mixture of the protective agent composition plus a biological sample; and a quenching agent in an amount sufficient to react with any free aldehyde (e.g., formaldehyde) that may arise from the imidazolidinyl urea to form a reaction product that will not react to denature any protein of the biological sample. The protective agent composition (prior to contact with any biological sample) may include from about 20% to about 60% by weight imidazolidinyl urea. The protective agent composition may include at least about 30% by weight imidazolidinyl urea. The protective agent composition may include at least about 40% by weight imidazolidinyl urea and less than about 55% by weight imidazolidinyl urea. The protective agent composition may include from about 1% to about 10% by weight of the quenching agent. The protective agent composition may include at least about 2% by weight of the quenching agent. The protective agent composition may include at least about 4% by weight of the quenching agent and less than about 8% by weight of the quenching agent. The protective agent composition may include from about 1% to about 20% by weight EDTA. The protective agent composition may include at least about 5% by weight EDTA. The protective agent composition may include at least about 7% by weight EDTA and less than about 10% by weight EDTA.

The protective agent composition may be pre-loaded into a tube and may be pre-loaded in amount of from about 50 to about 400µl of protective agent composition. The pre-loaded amount may be at least about 100µl and less than about 300µl. The pre-loaded amount may be at least about 150µl and less than about 250µl. Within the pre-loaded protective agent composition, the protective agent composition may comprise at least about 80 mg and less than about 100 mg of the protective agent composition. The quenching agent may comprise at least about 1 mg and less than about 15 mg of the protective agent composition. EDTA may comprise at least about 10 mg and less than about 25 mg of the protective agent composition. For the protective agent composition, it may include an amount of about 10 parts by weight of the protective agent composition per about 1 parts by weight of the quenching agent. The quenching agent may include a compound that includes at least one functional group capable of reacting with an electron deficient functional group of formaldehyde (e.g., an amine compound that reacts with formaldehyde to form methylol or imine Schiff base or a cis-diol compound that reacts with formaldehyde to form a cyclic acetal). The quenching agent may be an ingredient selected from amino acids, alkyl amines, polyamines, primary amines, secondary amines, ammonium salts, or a combination thereof. It may be an ingredient selected from glycine, lysine, ethylene diamine, arginine, urea, adinine, guanine, cytosine, thymine, spermidine, or any combination thereof. It may be an ingredient selected from glycine, lysine, ethylene diamine, urea or any combination thereof. The quenching step may include reacting any free aldehyde (e.g., formaldehyde) for forming a methylol, imine Schiff base, a Schiff base-quencher crosslink reaction product, a Schiff base dimer, or any combination thereof.

The isolation of cfDNA from plasma may include isolating cfDNA in the absence of any cell. Either or both of the isolating or analyzing steps may occur at least 2 hours, 7 days, or 14 days post venipuncture. Either or both of the isolating or analyzing steps may occur without and/or prior to any freezing of the sample or any of its constituents (i.e., to a temperature colder than about -30 °C. more preferably colder than about -70 °C)).

The protective agent composition optionally may include a nuclease inhibitor selected from the group consisting of: diethyl pyrocarbonate, ethanol, aurintricarboxylic acid (ATA), formamide, vanadyl-ribonucleoside complexes, macaloid, ethylenediamine tetraacetic acid (EDTA), proteinase K, heparin, hydroxylamine-oxygen-cupric ion, bentonite, ammonium sulfate, dithiothreitol (DTT), beta-mercaptoethanol (BME), cysteine, dithioerythritol, tris(2-carboxyethyl) phosphene hydrochloride, a divalent cation such as Mg²⁺, Mn²⁺, Zn²⁺, Fe²⁺, Ca²⁺, Cu²⁺, and any combination thereof. The protective agent composition may include a preservative composition, an aldehyde quenching agent, and an anticoagulant. In one preferred embodiment, the protective agent composition may include imidazolidinyl urea, glycine, and ethylenediamine tetraacetic acid.

The compositions and methods herein make possible the avoidance of essentially any free formaldehyde (or other aldehyde) in the sample, and thus helps to avoid any of the potentially deleterious effects that such free formaldehyde (or other aldehyde) may have upon nucleic acids (e.g., denaturation of proteins, undesired covalent bonding (such as between strands of DNA) or both, or some other chemical alteration of the cfDNA. The compositions and methods herein make possible the ability to amplify nucleic acid (e.g., DNA) samples that have been treated in accordance with the present teachings.

A determination herein that a composition is substantially devoid of free aldehyde (and particularly devoid of any free formaldehyde (which may be hydrated formaldehyde)) and/or methylene glycol may be performed using known techniques, such as by ¹³C nuclear magnetic resonance (NMR). For instance it may be performed by ¹³C nuclear magnetic resonance (i) in a deuterium oxide solvent, (ii) assisted by a relaxation agent (e.g., gadopentetic acid (Gd-DTPA)) or both (i) and (ii). A composition that is substantially devoid of free aldehyde (and particularly devoid of any free formaldehyde) and/or methylene glycol will typically exhibit no peak in a ¹³C NMR spectrum in the range of about 82-85 ppm.

Various concentration of preservative composition and quenching agent that may be contained within the protective agent composition may be also be analyzed using ¹³C NMR to determine which concentrations result in reduced amounts of free aldehyde as desired. More specifically, various preservative compositions (including imidazolidinyl urea (IDU), diazolidinyl urea (DU), nuosept 145 (methanol, formaldehyde), and oxaban-A (4,4-Dimethyl-1,3-oxazolidine) at various concentrations were combined with varying concentrations of a quenching agent (including glycine). For sample preparation, 16.7 mg Gd-DTPA was transferred into an empty vial and 3.5 mL of D₂O was added to the vial. Each sample was prepared by weighing an empty tube into which 15-17µl of THF was transferred and the weight of the tube with THF recorded. 300µL of sample solution in H₂O was transferred followed by the addition of 200µl of GD-DTPA/D₂O solution. The ¹³C NMR spectra were then acquired. Table 1 below depicts the results.

**Table 1**

| Sample ID | Preservative composition cone. | Glycine (%) | Protective agent composition amount (mg) (in 300µl NMR solution) | Formaldehyde (mg) (in 300µl NMR solution) | Cone. Of formaldehyde in original solution (%) |
|---|---|---|---|---|---|
| 1 | IDU (1%) | 0 | 3 | 0.0927 | 0.031 |
| 2 | IDU (1%) | 0.1 | 3 | 0.0799 | 0.027 |
| 3 | IDU (1%) | 0.2 | 3 | 0.0665* | 0.022* |
| 4 | DU (0.5%) | 0 | 1.5 | 0.1677 | 0.056 |
| 5 | DU (0.5%) | 0.05 | 1.5 | 0.1149 | 0.038 |
| 6 | DU (0.5%) | 0.1 | 1.5 | 0.0583 | 0.019 |
| 7 | Nuosept 145 (0.4%) | 0 | 1.2 | 0.1536 | 0.051 |
| 8 | Nuosept 145 (0.4%) | 0.04 | 1.2 | 0.1299 | 0.043 |
| 9 | Nuosept 145 (0.4%) | 0.08 | 1.2 | 0.0742 | 0.025 |
| 10 | oxaban-A (0.3%) | 0 | 0.9 | 0.0940 | 0.031 |
| 11 | oxaban-A (0.3%) | 0.03 | 0.9 | 0.0773* | 0.026* |
| 12 | oxaban-A (0.3%) | 0.06 | 0.9 | 0.0534* | 0.018* |
| *Values overestimated due to significantly low signal to noise ratio. | | | | | |

Any determination that a chemical alteration of the double helix formation of DNA has occurred may likewise be performed using known techniques such as spectrometry (e.g., a nucleic acid stain mediated spectrometry technique, such as SYBR green dye mediated fluorescence spectrometry), which may be employed to detect an optical phenomena such as fluorescing. With such an approach, DNA from a control sample (e.g., native DNA) may be measured and compared with a DNA from a treated sample to ascertain any effect on helical conformation, using circular dichroism (CD) spectra. It is expected that samples treated in accordance with the present teachings will exhibit a CD spectra that is generally in conformance with that of the native DNA, with an absence of indication of alteration of secondary structures.

A resulting mixture that has been treated in accordance with the present teachings may include one or more reaction products that include a methylol, Schiff base, a Schiff base-quencher cross-linked structure, or a Schiff base dimer. For example, for one illustrative composition, the teachings herein contemplate the presence (with or without a biological sample) of a combination of two or more of IDU, an IDU glycine reaction product, glycine, glycine methylol, a glycine Schiff base, a glycine cross-linked Schiff base, or a Schiff base dimer.

A resulting mixture that has been treated in accordance with the present teachings may include blood cells that are free of denaturation of cellular proteins. Resulting cell membranes may be such that the contents of the cells are able to be held within the membrane during processing and shipping with no detectable leakage. Accordingly, the sample may be of sufficiently high integrity that the cell-free fluids truly and accurately represent extra-cellular fluid. Further, though it may be possible that some combination with proteins (not nucleic acids) may occur, the combination is not irreversible, but rather can be removed by heating (e.g., to a temperature of about 60°C to about 90°C). Accordingly the teachings herein also contemplate an optional step of heating treated samples for a time and at a temperature sufficient to separate proteins (e.g., to a temperature of about 60°C to about 90°C). A heating step may be utilized as described herein for purifying a nucleic acid sample and removing any contamination from a nucleic acid sample. Such a heating step may be combined with the use of a protease which may be a serine protease such as proteinase K.

In one aspect, it is believed that the teachings herein unexpectedly are particularly useful for analysis of variable number of tandem repeat ("VNTR") analysis [not explicitly claimed]. This makes the teachings applicable for crime scene analysis, paternity testing, or other forensic DNA analysis. In such instances, it is possible that a method may employ drawing a blood sample of a subject into an evacuated blood collection tube that contains a protective agent composition in accordance with the present teachings. Blood cells accordingly can be stabilized for preventing release of their nucleic acid, and any free aldehyde (e.g., formaldehyde) quenched. Cell-free DNA can be isolated and optionally exposed to one or more suitable enzymes for cutting regions of DNA surrounding the VNTR's. Such VNTR's may be analyzed and compared with DNA collected from a crime scene. In the case of paternity testing, blood draw methods as above may be employed for both the offspring and the potential father of interest. Polymerase Chain Reaction (PCR) may be used for amplifying a sample. Resulting amplified samples may be analyzed, such as by suitable optical techniques for resolving the sample spectrally. For example, short tandem repeat (STR) loci may be multiplexed. It is believed that a surprisingly large amount of the sample is unaffected by the protective agent compositions herein, as demonstrated by there being an absence of denaturation of the STR loci and an absence of cross-linking at the loci. As such, a relatively high degree of confidence about the sample is achieved. Moreover, due to the relatively long-term stabilizing effects of the compositions (e.g., at least 1 week, 2 weeks, 4 weeks or longer), it makes it possible to use the sample over a prolonged period of time, and avoid the need for multiple blood draws.

The teachings herein are believed useful for analysis of one, two, three, five, eight, ten or thirteen or more of the following STR Loci: ACTBP2 (SE33) ; Amelogenin (X or Y); CD4; D2S1338; D3S1358; D3S1359; D7S809; D7S820; D8S347; D8S1179; D11S554; D12S391; D13S308; D13S317; D16S639; D18S51; D19S433; D21S11; F13A1; F13B; FABP; FES/FPS; FGA (FIBRA); FOLP23 (DHFRP2); HPRTB; LPL (LIPOL); TH01; TPOX; CSF1PO; D5S818; or VWA (vWF).

Data obtained about the DNA of a subject may be stored for subsequent retrieval, such as in a DNA database (e.g., the Combined DNA Index System (CODIS)). Subsequent cross-comparison of DNA profiles may be made with such information. DNA information may be employed for solving unsolved or "cold" cases (e.g., unsolved cases), for solving property crimes, for identifying persons or victims, or for some other purpose. One or more sample containers having a protective agent composition of the present teachings pre-loaded (e.g., in an evacuated blood collection device with a protective agent composition of the present teachings) may be provided as part of a forensic analysis kit. Any of the kits herein may include one or more additional components adapted for amplification and typing. It may include one or more sets of suitable primers (e.g., fluorescently labeled primers), which may be suitably adapted for short tandem repeat (STR) analysis. It may include sample containers (e.g., as described in Application Serial No. PCT/US2012/34506) for performing point of collection PCR analysis (e.g., with a portable thermocycler instrument such as the Philisa^{®} Instrument sold by Streck, Inc., or an instrument otherwise described in Application Serial Nos. PCT/US2012/040201, U.S. Application No. 13/484,963 (U.S. Application Publication No. 2012/0308990 A1), and U.S. Application Publication No. 2009/034446. Multiplexing polymerase chain reaction may be employed, which may involve adding one or more set of PCR primers (e.g., fluorescently labeled primers) to a sample being analyzed in order to selectively target a plurality of locations throughout the genome.

The teachings herein contemplate other applications, including but not limited to extracting cell-free DNA for use in detecting cancer (including but not limited to carcinomas, leukemia, and/or lymphoma). For instance, the teachings herein may be employed for detecting abnormal methylation for breast cancer, prostate cancer, gastric cancer, ovarian, colorectal cancer, bladder cancer, testicular cancer, esophogeal cancer, melanoma or other cancers. These methods are not literally claimed.

In accordance with the teachings, a blood sample may be contacted with a protective agent composition herein, any aldehyde may be quenched, and the resulting DNA extracted for analysis. The analysis may include analyzing methylation patterns of the DNA. The analysis may be employed for identifying methylation-based biomarkers (e.g., in one or more GC-rich fragment) associated with one or more of cancer, a neurogenerative disorder (e.g., Alzheimer's, Parkinson's disease, epilepsy, multiple sclerosis or otherwise), and/or a psychiatric disorder (e.g., schizophrenia, depression or otherwise). For example, the analysis may be employed for identifying the occurrence of methylated cytosine. The analysis may be employed for detecting, and/or monitoring viral infection, autoimmune diseases, microchimerism, acute myocardial infarction, stroke, mesenteric ischemia, and pancreatitis. Of course, the analysis may also be employed for pre-natal diagnosis (e.g., for one or more conditions such as an abnormal trisomy 13, trisomy 18, or trisomy 21 pathology, Klinefelter syndrome, Turner's syndrome, or pre-natal sex determination).

The analysis may employ amplification and detection by PCR, mass spectroscopy, parallel DNA sequencing or some other suitable analytical technique by which a disease state or condition is detected, diagnosed, treated and/or monitored.

The teachings herein also contemplate the possible employment of steps of monitoring therapies of a patient in response to some treatment (e.g., radiation and/or drug therapy). Blood samples may be taken at a first time, treated according to the teachings, and DNA analyzed. Blood samples may be taken at one or more times after the first time, treated according to the teachings, and DNA analyzed. Results of the respective analyses may be compared, and therapy may be altered in response to the result comparison.

The teachings herein may further be utilized to protect biological samples during shipping and storage. Transportation of blood samples from the site of phlebotomy to another facility is commonly required for molecular diagnostic testing. As discussed herein, several pre-analytical variables may compromise the accuracy cfDNA measurements, including the selection of blood collection devices and sample storage and shipping conditions. Each of these parameters affects the amount of nucleated blood cell lysis that occurs post-phlebotomy. Nucleated cell lysis leads to release of gDNA, elevating pDNA backgrounds and suppressing true cfDNA measurement. It is therefore desirable to minimize background gDNA increases that generally occur during shipping and storage based on sample movement and temperature changes.

During transportation, shaking may disrupt nucleated blood cell integrity and compromise accuracy, as described above. In simulated shipping conditions (using an orbital shaker) over a 24 hour period, blood samples were located in K₃EDTA and protective agent composition blood collection devices for comparison. An increase in pDNA concentration was observed at 6 and 24 hours in K₃EDTA blood samples. The protective agent composition stabilized blood cells and showed no considerable increase in pDNA under shaking conditions.

When samples were shipped, similar trends were observed as to when samples were shaken. Samples were shipped in either K₃EDTA or protective agent composition blood collection devices and the resulting pDNA concentration between the two tubes was compared. The protective agent composition samples showed stable pDNA concentrations before and after shipping, whereas K₃EDTA showed increases in pDNA under shipping conditions. This suggests nucleated cell disruption occurred in blood samples that were shipped in K₃EDTA leading to gDNA release, but this did not occur in the protective agent composition samples.

In current practice, blood samples are generally centrifuged to isolate plasma and frozen to prevent the gDNA contamination of cfDNA during sample processing, transportation and storage. The stabilizing chemicals within the protective agent composition prevent the release of gDNA into plasma post-phlebotomy up to 14 days, avoiding these labor-intensive requirements. Using the blood collection device disclosed herein, *ex vivo* storage at room temperature becomes possible, allowing flexibility for offsite blood draws to be sent to central laboratories for downstream analysis of the cfDNA without preliminary centrifugations or cryopreservation.

Samples come from or be in the initial form of one or more biological matter (i.e., urine, saliva, feces, synovial fluid, cerebrospinal fluid, or peritoneal fluid). Samples may include cells, or may be free of cells. In general, the samples are expected to have some initial amount of cell-free DNA, as to which the desire is to substantially preserve the amount of such cell-free DNA and to protect the cell-free DNA from deoxyribonuclease activity, so that it can be effectively analyzed.

Samples may be collected in any of number of ways. They may be collected as part of a venipuncture draw, by syringe, by a swab, by discharge collection, or otherwise. They may be collected in a container (e.g., a vial, a specimen collection cup, a collection tube, a capillary tube, or otherwise). The container may have a protective agent composition of the present teachings contained therein. The samples may be blood samples, and the methods may include obtaining a freshly drawn blood sample into an evacuated blood collection tube that includes the protective agent composition pre-loaded therein.

The methods may include one or more analytical step for analyzing the nucleic acids. For example, the methods may include a step of subjecting the sample to qualitative polymerase chain reaction amplification, quantitative polymerase chain reaction amplification or both after the quenching step. The methods may include a step of subjecting the sample to qualitative polymerase chain reaction amplification, quantitative polymerase chain reaction amplification or both after the quenching step that takes place at least four days, one week, or two weeks after the quenching step. The method may include a step of subjecting the sample to mass spectrometry. The method may include a step of subjecting the sample to mass spectrometry that takes place at least four days, one week, or two weeks after the quenching step. The method may include a step of subjecting the sample to DNA sequencing. The method may include a step of subjecting the sample to DNA sequencing that takes place at least four days, one week, or two weeks after the quenching step. The method may alternatively include a step of performing flow cytometry analysis on the sample.

Also advantageously envisioned within the teachings are methods of performing polymerase chain reaction amplification of a sample, comprising amplifying a nucleic acid (e.g., deoxyribonucleic acid (DNA)) from a biological sample that has been treated according to the above methods. Additionally, it is advantageously envisioned that a method of identifying variable number tandem repeats (VNTR) on a DNA sample, comprising treating a sample according to the teachings herein; determining a sample VNTR pattern for the sample; comparing a first VNTR pattern from a known DNA source with the VNTR pattern from the sample; and determining whether the sample VNTR pattern matches the first VNTR pattern.

The teachings herein may be utilized for any of a number of applications, and may be employed for enhancing the teachings of U.S. Published Application No. 2010/0184069 which includes genome wide screens of fetal DNA. For example the teachings herein may find suitable application for quantitative real-time PCR for genetic identification of Trisomy 21 (Down syndrome), pursuant to which there is at least one step of detecting an increase in DNA copy number (that is, a chromosomal duplication event within the fetal genome). As with analysis of samples according to the teachings herein, in general, this may be conducted at an off-site clinic (e.g., a location that is remote from the location of maternal blood draw (e.g., by at least 100 meters, 1000 meters, 10,000 meters or further)). Due to the remote nature, there will typically be a step of transporting the sample from the draw site to a clinical analysis site. Such transport (again, as with analysis of samples according to the teachings herein, in general) may be without refrigeration. These methods are not literally claimed.

Moreover, as indicated, the teachings provide for useful application in forensic DNA analysis. By way of illustration, for forensic analysis, VNTR DNA may employ using primers flanking the same VNTR regions, in the course of amplifying a DNA sample. Amplified VNTRs may be collectively run on an individual lane of an agarose gel, which resolves the VNTR DNA segments based upon their size and specific to a subject's (e.g., a crime suspect's) known VNTR pattern. Qualitative comparisons of the DNA size patterns on the agarose gel may be compared to a DNA sample collected at a crime scene for match. The teachings herein may be employed for obtaining and preserving a suspect's blood sample, for obtaining and preserving a crime scene sample, or both. These methods are not literally claimed.

### Examples

Blood samples were drawn from healthy donors into K₃EDTA and blood collection tubes containing a protective agent composition. To simulate shipping conditions, samples were either shaken or unshaken. In a shipping study samples were either shipped or not shipped. To assess temperature variations, samples were incubated at 6 °C, 22 °C and 37 °C. In all cases plasma was harvested by centrifugation and total plasma DNA (pDNA) assayed by quantitative real-time PCR (qPCR). Shaking blood in K₃EDTA tubes showed significant increases in pDNA whereas no change was seen in the protective agent composition samples. Blood shipped in K₃EDTA tubes showed significant increases in pDNA versus those shipped in the protective agent composition. Blood in K₃EDTA tubes incubated at 6 °C, 22 °C and 37 °C showed significant increases in pDNA while pDNA from the protective agent composition samples remained stable. The protective agent composition prevents increases in background gDNA levels that can occur during sample storage and shipping. Thus, the protective agent composition provides a novel way to obtain high quality stabilized samples for low abundance DNA target detection and accurate cfDNA concentrations.

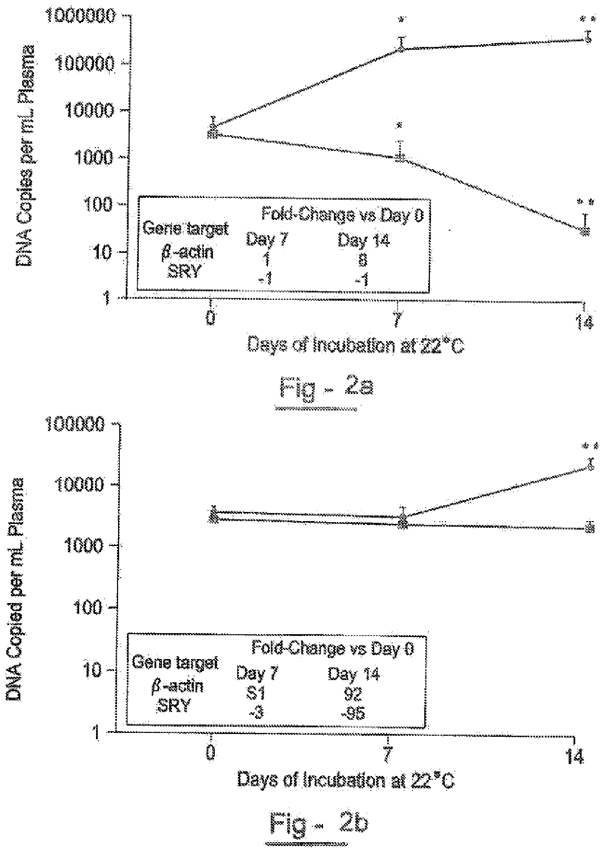

PCR detection of cell-free DNA targets within a high gDNA background is challenging, requiring specialized protocols and/or large volumes of starting material. As a result, minimizing the release of gDNA from nucleated cells is essential for accurate analysis true cfDNA. Using the protective agent composition it is possible to preserve the original proportion of fetal cfDNA by minimizing maternal gDNA background. Increased background gDNA adversely affects the detection of low abundance cfDNA targets (Fig. 2C). As pDNA concentration increased on days 7 and 14 in K₃EDTA samples due to gDNA release, detection of the introduced SRY fragments decreased (Fig. 2a). In contrast, there was no significant change in pDNA concentration because of gDNA release in the protective agent composition samples at day 7 and minimal gDNA release on day 14, but detection of SRY fragments remained steady throughout the entire time period (Fig. 2b).

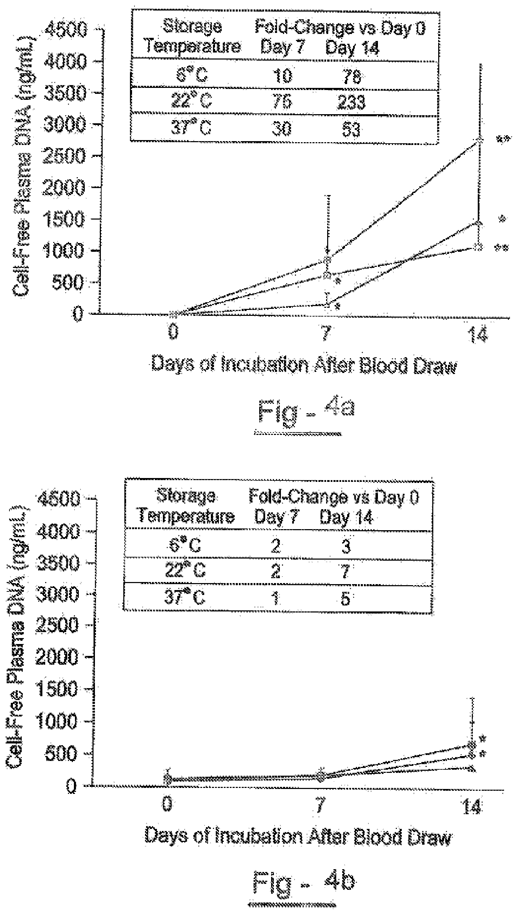

Variation in sample storage temperature is another post-phlebotomy condition that can cause changes in gDNA concentration. The effect of three different storage temperatures on the pDNA concentration of blood samples drawn into K₃EDTA and the protective agent composition was monitored. Following blood draw, samples were incubated at 6°C, 22°C or 37°C for 14 days. Significant increases in K₃EDTA blood sample pDNA concentrations were seen at 6°C and 37°C by day 7 and at all temperatures by day 14 (Fig. 4A). Blood drawn into the protective agent composition tubes showed no increase in pDNA concentration at any temperature on day 7 and showed statistical changes in pDNA concentration at day 14 when incubated at 6°C and 22°C (Fig. 4B). However, fold change calculations comparing K₃EDTA and the protective agent composition samples pDNA levels on day 0 versus day 7 and day 14 (see Figs. 4A and 4B). This demonstrates the ability of the protective agent composition to stabilize pDNA levels and prevent gDNA release across a broad temperature range for an extended period of time.

Blood donors were recruited with informed consent from Streck, Inc. in Omaha, NE. Donors were both male and female and presumed to be healthy. All draws were performed using venipuncture. For each experiment, donor samples were drawn into two different blood collection tubes. Control samples were drawn into K₃EDTA tubes (BD Vacutainer^{®}, Becton Dickinson, Franklin Lakes, NJ) and compared to samples drawn into the protective agent composition described herein. Blood was mixed immediately after the draw by inverting 10 times.

### Sample processing

After phlebotomy blood samples were stored at room temperature (22°C), except where otherwise noted, and plasma was separated at noted time points. For the separation of plasma, blood samples were centrifuged at 22°C at 300 × g for 20 minutes. The plasma layer was carefully removed without disturbing the buffy coat, transferred to a new vial using a pipette and then centrifuged at 22°C at 5000 xg for 10 minutes to remove residual cells.

### Cell-free DNA isolation from plasma

The QIAamp^{®} Circulating Nucleic Acid Kit (Qiagen, Santa Clarita, CA) was used for the extraction of pDNA. The manufacturer's recommended protocol was modified slightly by increasing the duration of the Proteinase K treatment from 30 minutes to 1 hour at 60°C. Samples were eluted in 50 µL sterile nuclease-free water and stored at -80°C until analysis by real-time qPCR.

### Quantitative Real Time PCR (qPCR)

Human β-actin primers and probe and primers for the human Y-chromosomal sex determining region (SRY) were prepared as previously described by Chan et al. (Chan KC, Ding C, Gerovassili A, et al. (2006) Hypermethylated RASSF1A in maternal plasma: A universal fetal DNA marker that improves the reliability of noninvasive prenatal diagnosis. Clin. Chem 52:2211-2218) and by Lee et al. (Lee TH, Paglieroni T, Ohto H, et al. (1999) Survival of donor leukocyte subpopulations in immunocompetent transfusion recipients: frequent long-term microchimerism in severe trauma patients. Blood 93:3127-3139.) The following probe was designed for the quantification of SRY sequence: 6FAM-ATG GCT CTA GAG AAT CCC AGA ATG CGA AAC TCA GAG A-TAMRA. For each assay, 10-fold dilutions (300,000-30 copies) of plasmid DNA constructs were used to establish qPCR standard curves. Constructs were prepared by cloning a single copy of β-actin or SRY DNA sequence, which produced amplicons of 136 bp or 148 bp, respectively. β-actin genomic equivalents were calculated from qPCR copy number and then converted to nanograms of cfDNA per milliliter plasma (ng/ml). The final SRY DNA concentration was expressed as DNA copies recovered per milliliter (copies/ml) plasma. All primers were purchased from Integrated DNA Technologies (Coralville, IA). The qPCR probes and TaqMan^{®} Universal PCR Master Mix II were purchased from Applied Biosystems (Foster City, CA).

### Effect of storage temperature on pDNA concentration in blood samples

Blood was collected from eight donors into K₃EDTA tubes and the protective agent composition tubes. Blood was aliquoted and then divided into three sets comprised of three K₃EDTA and three protective agent composition aliquots each. One set of aliquots was stored at 6°C, another set at 22°C and the last set at 37 °C. Plasma was harvested at each temperature on day 0, 7 and 14 and stored at -80°C until pDNA was extracted and analyzed by qPCR using the β-actin assay.

### Effect of shaking and shipping on pDNA concentration in blood samples

To simulate shipping blood from eight donors was drawn into K₃EDTA tubes and the protective agent composition tubes. Tubes were secured onto the platform of an orbital shaker and shaken at 150 rpm at 22°C. At 0, 3, 6 and 24 hour time points, plasma from one K₃EDTA and one protective agent composition tube was harvested and frozen at -80°C until extraction and analysis. A control experiment was done where the blood was not shaken while all other experimental parameters remained the same. In a separate shipping study, blood from ten donors was drawn into K₃EDTA tubes and the protective agent composition tubes. Tubes were shipped round trip via air freight to a lab in Springfield, MA (elapsed time was 4 days) and plasma was harvested upon return stored at -80°C until extraction and analysis. A control set of tubes were not shipped and plasma harvested was on day 0 and 4. For both shaken and shipped samples, pDNA was measured by qPCR using the β-actin assay.

### Statistical analysis

Statistical analysis was carried out using the Tools for Science website of the College of Saint Benedict Physics Department, Saint John's University, St. Joseph, MN, USA (http://www.physics.csbsju.edu/). Analysis was performed using unpaired Student's t-test and *p* < 0.05 was considered statistically significant.

### Effect of chemicals present in the protective agent composition on pDNA amplification

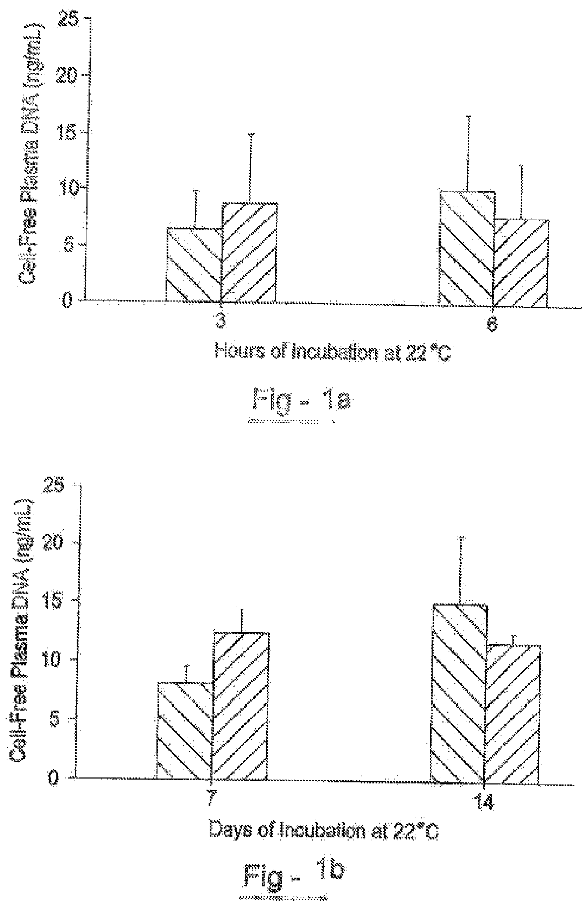

We investigated the effect of the protective agent composition on pDNA amplification by qPCR (Fig. 1). Blood was drawn into K₃EDTA tubes and plasma was separated by centrifugation. The plasma supernatant was aliquoted and divided into two treatment groups. To one group of plasma samples (gray bars), the protective agent composition was added at a final concentration equal to the amount present in a standard 10 mL blood draw. The other group of plasma samples (black bars) was untreated and served as a control. Samples from each group were processed at 3 and 6 hours (panel a) and at 7 and 14 days (panel b). The pDNA concentration was determined using the β-actin qPCR assay. When compared to untreated samples, samples treated with the chemicals showed no decrease in either DNA extraction from plasma or its amplification by qPCR. Error bars indicate SD, *n* = 5 for both panels. In Fig. 1, comparison of pDNA concentrations in chemically treated and untreated plasma samples showed no decrease in amplification after 3 and 6 hours of incubation at 22°C (Fig. 1A, p = 0.44 and *p* = 0.52, respectively); samples incubated for 7 and 14 days showed similar results (Fig. 1B, p = 0.0036 and *p* = 0.23, respectively). These findings indicate the chemicals present in the protective agent composition had no adverse effects on either DNA extraction from plasma or its amplification by qPCR.

### Effect of elevated background gDNA in plasma on detection of rare cfDNA sequences

We simulated the effect of increased gDNA concentration, measured by the β-actin assay, on the detection of low abundance cfDNA targets by introduction of a male-specific DNA (SRY) into non-pregnant female blood (Fig. 2). Blood was drawn from non-pregnant female donors into K₃EDTA and protective agent composition tubes and stored at 22°C for 0, 7 or 14 days. At each time point, plasma was separated by centrifugation. A plasmid DNA construct containing a SRY sequence fragment (3000 copies) was then spiked into all plasma samples and pDNA was extracted. Total plasma DNA concentration was determined using the β-actin qPCR assay (●) and Y-chromosomal sequence copy numbers were determined by the SRY qPCR assay (■). For K₃EDTA samples (Fig. 2A), as β-actin pDNA concentrations increased in K₃EDTA samples (*p < 0.05, ^{∗∗}*p* < 0.001), there were significant decreases in SRY sequence copy number detection (^{∗∗}*p* < 0.001). For the protective agent composition samples (Fig. 2B), pDNA concentrations remained close the initial day 0 value (**p < 0.001) and the SRY sequence was detectible throughout the 14 day time course. Table insets in each figure show the fold change when comparing day 0 β-actin and SRY levels to those on days 7 and 14. Error bars indicate SD, *n* = 5 for both panels.

Figure 2a shows the DNA concentration of both gene targets in harvested plasma from K₃EDTA samples stored at 22 °C for 0, 7 and 14 days. As gDNA was released into plasma over time, significant increases in gDNA (β-actin) levels were observed on day 7 and 14, as compared to the initial concentration on day 0 *(p* = 0.0087 and *p* = 0.0002). As gDNA (β-actin) levels increased, detection of the cfDNA target (SRY fragment) significantly decreased (day 7, *p* = 0.0068 and day 14,p = <0.0001).

Figure 2b shows the pDNA concentration of the protective agent composition plasma samples stored under the same storage conditions as above. Plasma harvested from the protective agent composition samples showed no significant change in the β-actin concentration at 7 days (p = 0.55) but did show a statistical change at the 14 day time point (p = 0.0002). With the protective agent composition, gDNA (β-actin) levels remaining close to day 0 value, cfDNA (SRY fragment) levels did not change and remained detectible throughout the entire time course (day 7, *p* = 0.45 and day 14, *p* = 0.20).

In order to more clearly demonstrate how changes in gDNA levels effect cfDNA target detection, fold change calculations were performed (Fig. 2C). As K₃EDTA gDNA levels increased (β-actin, day 7 = 51-fold change, day 14 = 92-fold change), cfDNA detection decreased (SRY, day 7 = -3-fold change, day 14 = -95-fold change). The protective agent composition gDNA levels remained relatively steady (β-actin, day 7 = 1-fold change, day 14 = 8-fold change) as did the cfDNA target detection (SRY, day 7 = -1-fold change, day 14 = -1-fold change).

### Effect of shaking and shipping on pDNA concentration in blood samples

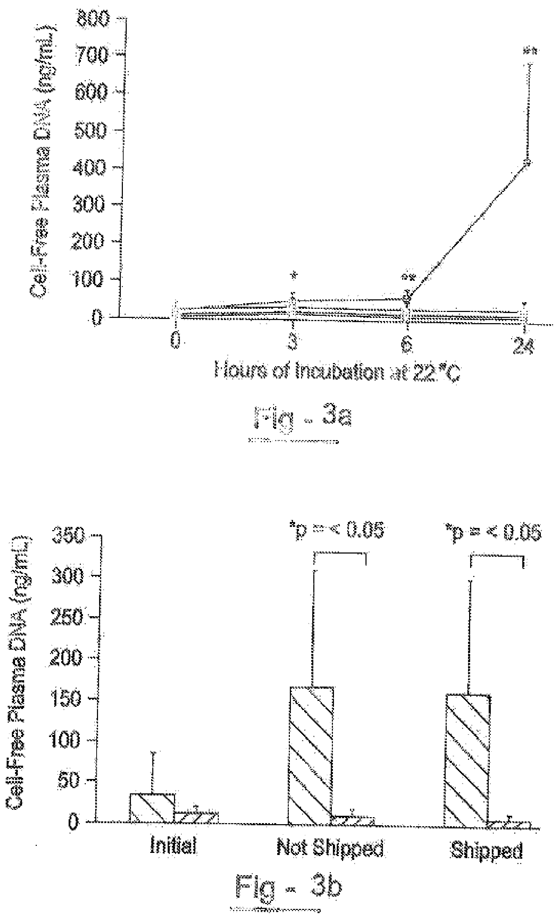

Agitation conditions present during sample transport were simulated with an orbital shaker. A control experiment was also performed using both types of tubes without agitation (Fig. 3). Blood was drawn into K₃EDTA tubes and the protective agent composition tubes and then agitated at 22°C on an orbital shaker. As shown in panel A, at times 0, 3, 6 and 24 hours, one K₃EDTA tube (o) and one protective agent composition tube (□) was removed and pDNA was isolated. A control experiment was also performed with blood drawn from the same donors into K₃EDTA tube (●) and protective agent composition tube (■) over the same time period without shaking. The pDNA concentration was determined using the β-actin qPCR assay. K₃EDTA tubes that were subjected to shaking showed statistically significant increases in pDNA concentration at 3, 6 and 24 hours compared to time 0 (^{∗}*p* < 0.05, ^{∗∗}*p* < 0.001). Total plasma DNA concentration remained stable in both shaken protective agent composition samples and unshaken samples. Error bars indicate SD, *n* = 8 in panel A. In a shipping study, as shown in panel B, blood was drawn into K₃EDTA tubes (black bars) and protective agent composition tubes (grey bars) and either shipped round trip to a lab in Springfield, MA or left not shipped. Upon return, plasma from shipped and not shipped samples was isolated and the pDNA concentration was determined using the β-actin qPCR assay. K₃EDTA tubes that were shipped showed a significant increase in pDNA when compared to shipped protective agent composition tubes (*p < 0.05). K₃EDTA tubes that were not shipped showed similar significance when compared to protective agent composition tubes that were not shipped. pDNA concentration in shipped and not shipped protective agent composition samples showed no statistical change when compared to time zero. Error bars indicate SD, *n* = 10 in panel B.

Figure 3a shows that shaking blood drawn into K₃EDTA tubes caused a significant increase in the pDNA concentration at 3, 6 and 24 hours post-blood draw (p = 0.031, 0.0003 and 0.001), whereas shaking blood drawn into the protective agent composition tubes showed no change (p = 0.14, 0.34 and 0.31). Unshaken control samples drawn into K₃EDTA and The protective agent composition tubes showed no change in pDNA concentration after 3 *(p* = 0.14 and 0.22), 6 *(p* = 0.33 and 0.52) or 24 hours incubation *(p* = 0.29 and 0.72).

After mimicking shipping, new samples were drawn into either K₃EDTA or the protective agent composition tubes. Tubes were either shipped to a lab in Springfield, MA and back over the course of four days or left not shipped at 22°C. Figure 3b illustrates a significant increase in pDNA concentration between shipped K₃EDTA and shipped protective agent composition samples (p = 0.01) after they had returned. There was also a significant increase in pDNA between initial and shipped K₃EDTA (p = 0.015) and between initial and not shipped K₃EDTA (p = 0.013). There was, however, no change in pDNA concentration between the initial and not shipped or the not shipped and shipped Protective agent composition tubes (p = 0.399 and 0.340).

### Effect of storage temperature on pDNA concentration in blood samples

To demonstrate the effect of temperature on pDNA levels in blood drawn into K₃EDTA tubes and protective agent composition tubes, samples were stored at either 6°C, 22°C or 37°C for up to 14 days (Fig. 4). Blood was drawn into K₃EDTA and the protective agent composition. Blood was aliquoted and then divided into three sets, each comprised of 3 K₃EDTA and 3 protective agent composition aliquots. One set of aliquots was incubated at 6°C (▲), one set at 22°C (●) and one set at 37°C (■). Plasma was separated by centrifugation from aliquots at each temperature on day 0, 7 and 14. The pDNA concentration was determined using the β-actin qPCR assay. Samples drawn into K₃EDTA (Fig. 4A) and stored at all temperatures showed statistically significant increases in pDNA concentration as compared to the day 0 value, while pDNA isolated from protective agent composition samples (Fig. 4B) showed minimal change during the 14 day period (^{∗}*p* < 0.05, ***p* < 0.001). Table insets on each figure show the fold change when comparing day 0 pDNA levels to those on days 7 and 14. Error bars indicate SD, *n* = 8 for both panels.

Figure 4a shows significant increases in pDNA concentration in K₃EDTA samples on days 7 and 14 when compared to initial values at 6°C (day 7, *p* = 0.039, day 14, *p* = 0.041) and on day 14 when stored at 22°C (day 7, *p* = 0.056, day 14, *p* = 0.0002). K₃EDTA samples incubated at 37 °C showed a high degree of hemolysis throughout the time course and the pDNA concentration at day 14 (day 7, *p* = 0.003, day 14, *p* = < 0.0001) was somewhat lower compared to samples incubated at 6°C and 22°C. No hemolysis was visible in protective agent composition samples when stored at 37°C. Blood samples drawn into the protective agent composition tubes and stored at 6°C, 22°C and 37°C showed no significant change in pDNA concentration at day 7 compared to the day 0 value (Figure 4B, p = 0.15, *p* = 0.32, and *p* = 0.61, respectively). Statistical changes were seen on day 14 in protective agent composition samples stored at 6°C *(p* = 0.048) and 22°C *(p* = 0.039), but not at 37°C *(p* = 0.070).

Fold change calculations (Fig 4a and 4b, table inset) illustrate the increase in K₃EDTA pDNA levels on day 7 and 14 (6°C = 10-and 78-fold increase, 22°C = 75- and 233-fold increase, 37°C = 30- and 53-fold increase, respectively), while changes in pDNA levels from The protective agent composition samples remained minimal (6°C = 2- and 3-fold increase, 22°C = 2- and 7-fold increase, 37°C = 1-and 5-fold increase, respectively).

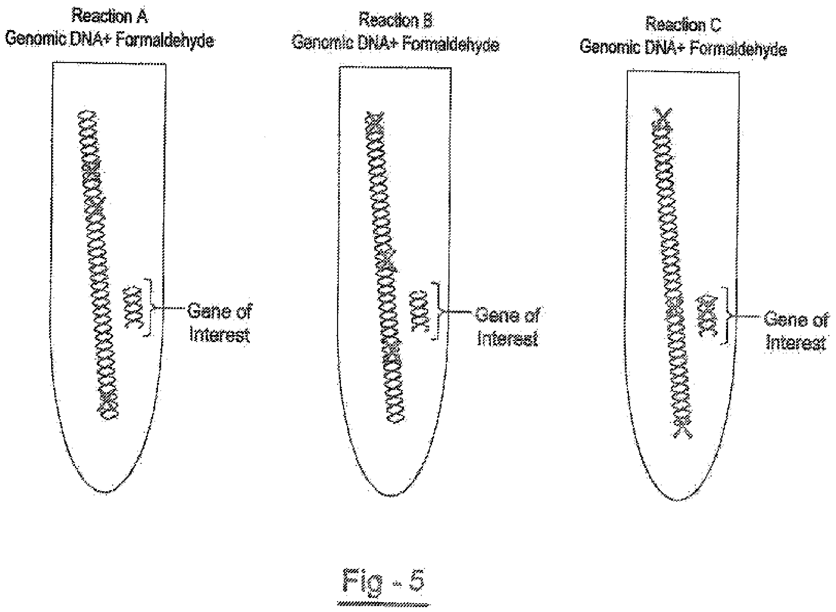

With reference to Fig. 5, formaldehyde is used as a cell fixative due to its ability to cross-link amino, amido, guanidino, thiol, phenolic, imidazolyl, and indolyl groups on nucleic acids and protein to form hemi-acetal derivatives. DNA-protein or DNA-DNA crosslinks are a specialized, but important, type of damage that blocks the genetic investigation of an enormous number of stored samples. The formalin-induced cross-linking effectively preserves DNA structural morphology, but it is extremely detrimental to subsequent DNA analysis because cross-linked bases stall polymerases and DNA-DNA cross-links can inhibit denaturation. Because the human genome is so large (3 billion deoxyribonucleotide pairs), it is impossible to determine which nucleotides will be cross-linked when exposed to a formalin solution (i.e.; hemi-acetal addition to DNTPs by formalin occurs by random chance). Therefore, detection of formalin-induced DNA damage using a single gene analysis is not sufficient. For example, Fig. 5 illustrates three different reactions containing the same preparation of genomic DNA and formaldehyde. However, the site of formaldehyde cross-linking is different in each tube.

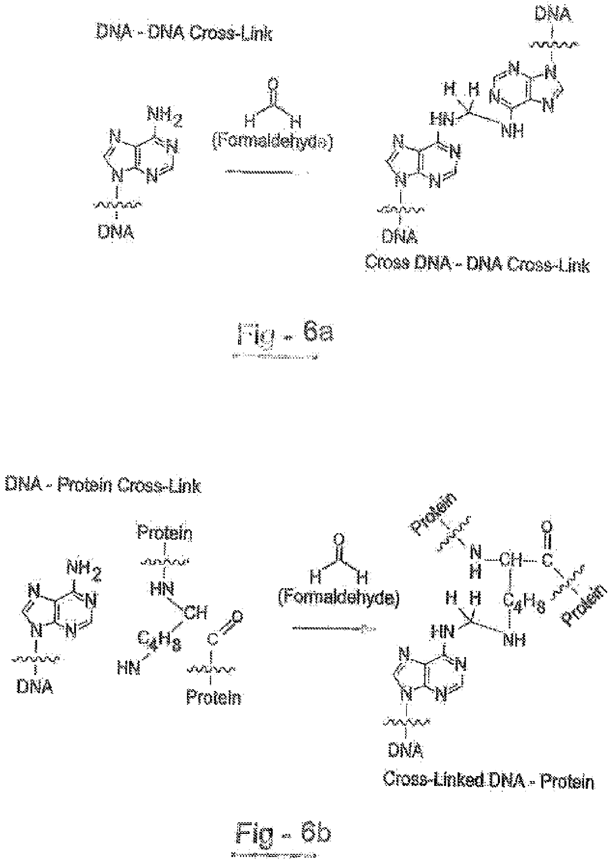

Figs 6a and 6b illustrate schematic diagrams of the chemical modifications of methylene bridge formation between two adenine nucleobases (an example of DNA-DNA cross-link), and between adenine nucleobase and lysine (an example of DNA-protein cross-link) that occur in DNA-DNA and DNA-protein cross-linking.

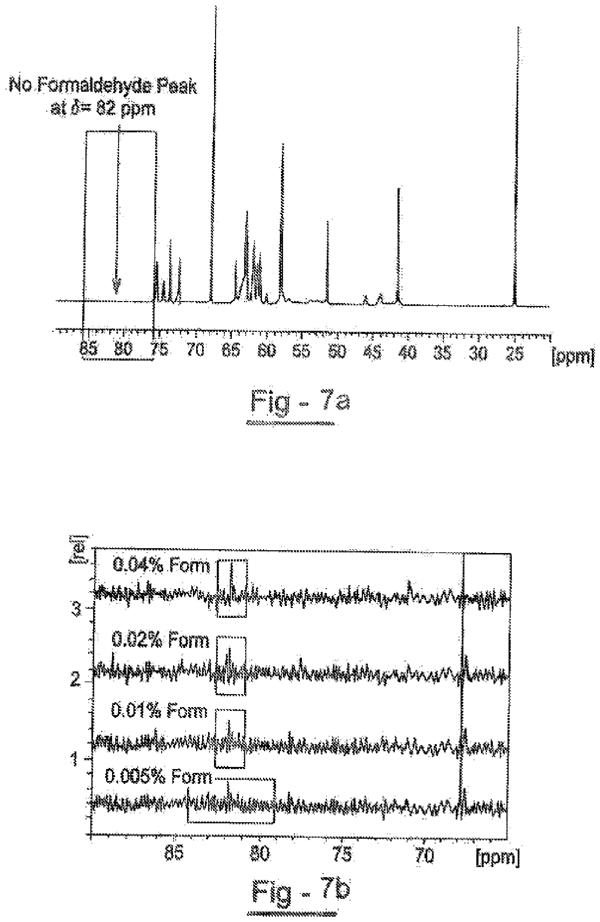

Figs. 7a and 7b illustrate how the teachings herein avoid detectable free formaldehyde. In the upper plot, a ¹³C NMR spectrum of cfDNA contacted with the protective agent composition shows no peak of formaldehyde (methylene glycol) around 82-85 ppm (shaded region). In the lower plot, there is shown a comparative example of ¹³C NMR peak of formaldehyde in formaldehyde solution (methylene glycol) at various concentrations. By way of illustration, one approach to determining the presence or absence of an aldehyde, such as formaldehyde, may employ quantitative ¹³C NMR analyses, carried out in the presence of gadolinium diethylenetriaminepentaacetate (Gd-DTPA) complex. The NMR data may be acquired by an instrument having relevant capabilities consistent with those of the 500 MHz Avance DRX series NMR spectrometer with TXI Cryoprobe, Bruker Biospin Corp (Billerica, MA). The NMR data may be processed by software having relevant capabilities consistent with that of the Bruker Topspin software. For obtaining NMR data, 5 mm outer diamter borosilicate NMR tubes (e.g., from New Era Enterprise, Inc, Vineland, NJ) may be used. A suitable spectrometer such as a Bruker spectrometer operating at 125 MHz ¹³C observation frequency at a probe temperature of 298 K may be employed. An inverse-gated proton decoupling sequence (zgig30) may be applied for the acquisition. Illustrative parameters for ¹³C{¹H}-NMR were spectral width -- 30030 Hz; time domain points -- 32768; acquisition time -- 0.5 second; pulse delay -- 1 second, pursuant to which a suitable number of scans (e.g., 2048 scans) may be acquired. Prior to each NMR experiment Gd-DTPA may be dissolved in D₂O to obtain the solution with desired concentration. For NMR acquisition, 250 µL sample solution may be diluted with 250 µL of Gd-DTPA/D₂O. THF may be added by weight directly into the NMR sample solution.

A conventional NMR chemical shift unit ppm (parts per million) may be used for spectral interpretations, with peaks calibrated based on -*CH*₂-CH₂O- of tetrahydrofuran (THF, an inert internal reference) peaks at □= 24.9 ppm. The NMR chemical shift unit ppm is the ratio of the difference between the chemical resonance of the analyte and the reference substances to the frequency of the instrument. It can be expressed by dimensionless quantity "0" and defined as: □□= [(□ _{Sample} - □_{Reference}) × 10⁶]/operating frequency.

To detect the lower detection limit of the NMR conditions, ¹³C NMR spectra of formaldehyde solutions with gradually decreasing formaldehyde concentrations may be acquired. In Figure 7b, for instance, the ¹³C NMR spectrum, with 2048 repeating scans and 1 second relaxation delay of 0.01% of formaldehyde solution, indicates the methylene glycol *CH₂* signal at ~81.9 ppm has a signal to noise ratio of nearly 2. A similar spectrum with 0.005% formaldehyde did not show presence of any peak in the 80-85 ppm region. Therefore, the lower detection limit of the applied NMR technique for detecting formaldehyde is between 0.01% and 0.005%. Fig. 7A shows the representative ¹³C-NMR spectrum of cell-free DNA BCT reagents (an exemplary protective agent composition within the present teachings) (250 µL) in D₂O (250 µL) in the presence of Gd-DTPA (~0.6 mg). The ¹³C NMR spectra of multiple lots of such compositions show no signal in the 80-85 ppm region. Similar results are believed achievable with samples treated in accordance with the present teachings. That is, not only is the protective agent composition free of detectable formaldehyde by ¹³C-NMR analysis, but samples treated with the composition are likewise free of detectable formaldehyde by ¹³C-NMR analysis.

Fig. 8a illustrates how conformational damage to DNA can be avoided using the teachings herein. There is depicted fluorescence spectra of native-DNA (control), CF-DNA-BCT (an exemplary protective agent composition within the present teachings) preserved DNA, formaldehyde preserved DNA and glutaraldehyde preserved DNA. The top plot is for a sample held at room temperature for 7 days. The middle plot is for a sample held at room temperature for 7 days and then heated at 60 °C for 1 hr. The lowest plot is for a sample held at room temperature for 7 days and heated at 90 °C for 2 min.

Fig. 8b further illustrates how conformational damage to DNA can be avoided using the teachings herein. There is depicted fluorescence spectra of native-DNA (control), CF-DNA-BCT (an exemplary protective agent composition within the present teachings) preserved DNA, formaldehyde preserved DNA and glutaraldehyde preserved DNA. The top plot is for a sample held at room temperature for 14 days. The middle plot is for a sample held at room temperature for 14 days and then heated at 60 °C for 1 hr. The lowest plot is for a sample held at room temperature for 14 days and heated at 90 °C for 2 min.

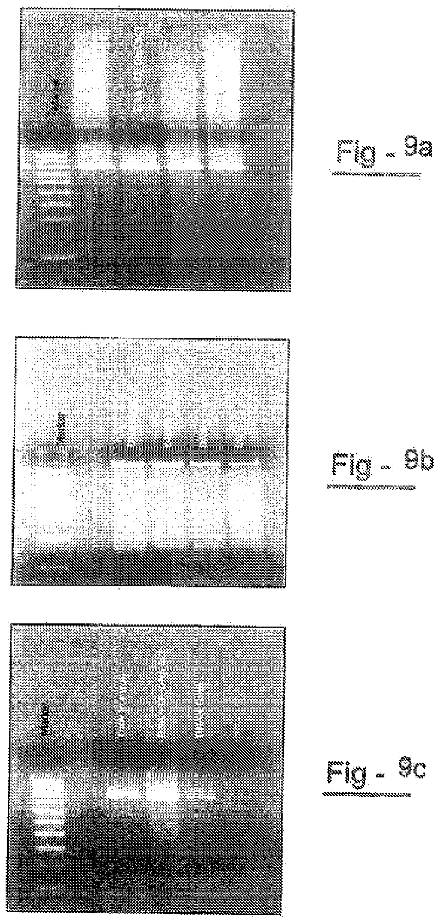

Fig. 9 illustrates how amplification of DNA samples can benefit from the teachings herein. DNA samples are incubated with various agents at room temperature. A DNA Control is employed. Treated samples are employed for comparison, including a CF-DNA BCT (an exemplary protective agent composition within the present teachings) treated sample, a formaldehyde (0.1%) treated sample and a glutaraldehyde (0.1%) treated sample for comparison. Aliquots are taken out from each DNA sample at various times and amplified by PCR.

Immediately after addition of the agents for treating the samples, per Fig. 9a, PCR amplifications are similar for all DNA samples mixed with different agents. PCR amplification does not appear affected by the presence of the fixatives immediately upon contact with each sample. However, about 18 hours after addition of agents, per Fig. 9b, PCR amplifications of control DNA and CF-DNA BCT treated DNA are similar. However, PCR amplification of formaldehyde fixed DNA is hindered, and glutaraldehyde fixed DNA is further hindered. It is thus believed that use of CF-DNA BCT (an exemplary protective agent composition as described herein) in accordance with the present teachings does not damage DNA; whereas formaldehyde and glutaraldehyde damage DNA.

After about 72 hours, per Fig. 9c, PCR amplification of control DNA and CF-DNA BCT treated DNA are similar; PCR amplification of formaldehyde fixed DNA is remarkably hindered; and glutaraldehyde fixed DNA did not amplify. Accordingly, it is again believed that over extended periods (greater than 24, 48 or 72 hours), CF-DNA BCT does not damage DNA; whereas formaldehyde and glutaraldehyde damage DNA.

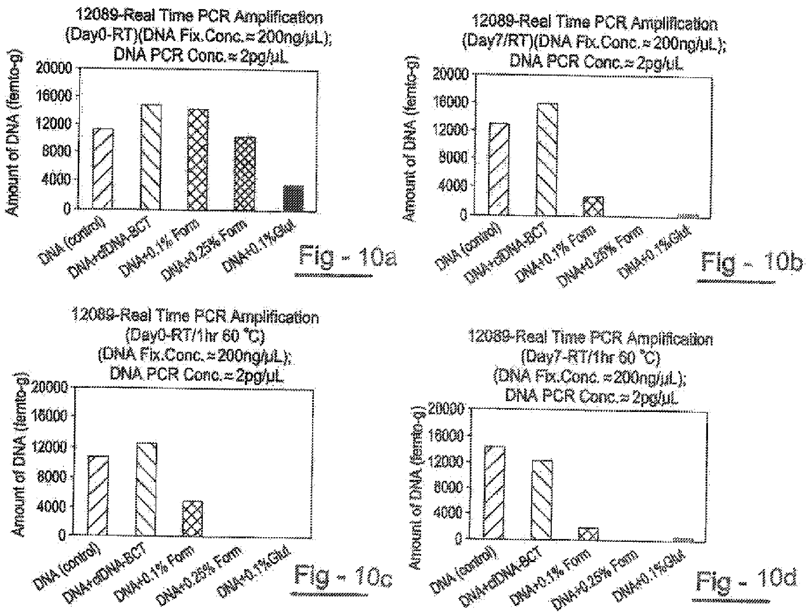

Figs. 10a-10d illustrate quantitative real-time PCR analysis of an 800 base pair gene fragment of human glyceraldehyde 3-phosphate dehydrogenase (GAPDH) DNA treated with or without formaldehyde, glutaraldehyde, or CF-DNA BCT reagent (an exemplary protective agent composition within the present teachings) at the indicated concentrations. Samples are maintained at room temperature (RT; a and b) or an additional heat treatment is done for 1 hour at 60 °C prior to PCR amplification (c and d). Quantification of starting DNA concentration, post-amplification, of GAPDH DNA fragments is performed on a Bio-Rad iQ5^{™} Thermocycler using the TaqMan Universal Master Mix II with UNG. Data presented represents 0 (a and c) and 7 (b and d) day time points. The results indicate that GAPDH DNA treatment with CF-DNA BCT (an exemplary protective agent composition within the present teachings) does not prevent PCR amplification of the GAPDH DNA and quantified DNA concentrations are comparable to the non-treated DNA control at time 0, indicating CF-DNA BCT reagent does not impair PCR analysis of the samples at 7 days post-sample draw. However, formaldehyde or glutaraldehyde treatments partially or completely impair the amplification of the GAPDH DNA. In summary, these data demonstrate that CF-DNA BCT reagent, but not formaldehyde or glutaraldehyde, can be used to stabilize and maintain DNA integrity for downstream quantitative analysis by quantitative PCR-based methodologies.

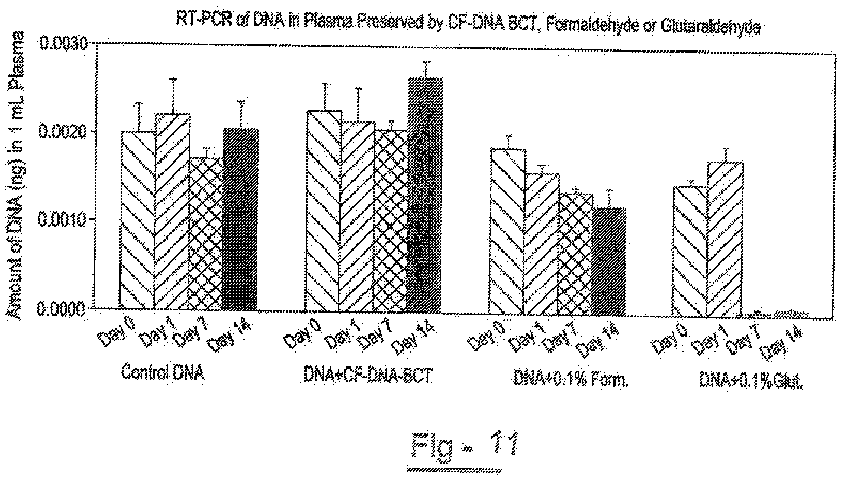

Fig. 11 illustrates the effects of different stabilization reagents on plasma cfDNA amplification. An *in vitro* model in which 800 base-pair DNA amplicon of human genomic DNA is spiked into plasma from healthy human blood. DNA spiked plasma samples were incubated with or without different stabilization agents at room temperature. Treated samples were employed for comparison, including a CF-DNA BCT (an exemplary protective agent composition within the present teachings) treated sample, a formaldehyde (0.1%) treated sample and a glutaraldehyde (0.1%) treated sample for comparison. Aliquots are taken out from each DNA sample at indicated times; DNA was extracted using Qiagen extraction kit and amplified by quantitative real-time PCR. Quantification of treated and untreated DNA was performed on a Bio-Rad iQ5^{™} Thermocycler using syber-green chemistry protocol. Fig 11 demonstrates that untreated DNA (control) and CF-DNA BCT treated DNA continue to amplify at a substantially similar extent; whereas both formaldehyde and glutaraldehyde treated DNA has continuously decreasing amplification over time.

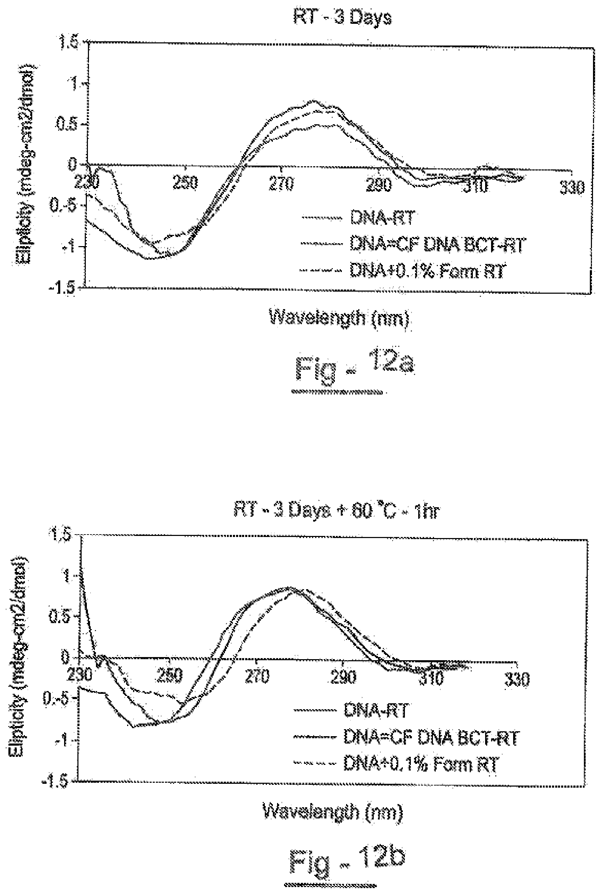

Fig. 12 illustrates DNA double helical conformation damage by formaldehyde after heating at 60 °C for 1 hour (this was done to stimulate the proteinase K digestion step which is done at 60 °C for 1 hour during the cfDNA extraction from plasma), but not with CF-DNA-BCT reagent according to the present teachings. Circular dichroism spectra of native-DNA (control), CF-DNA-BCT preserved DNA (an exemplary protective agent composition within the present teachings), and formaldehyde preserved DNA are shown.

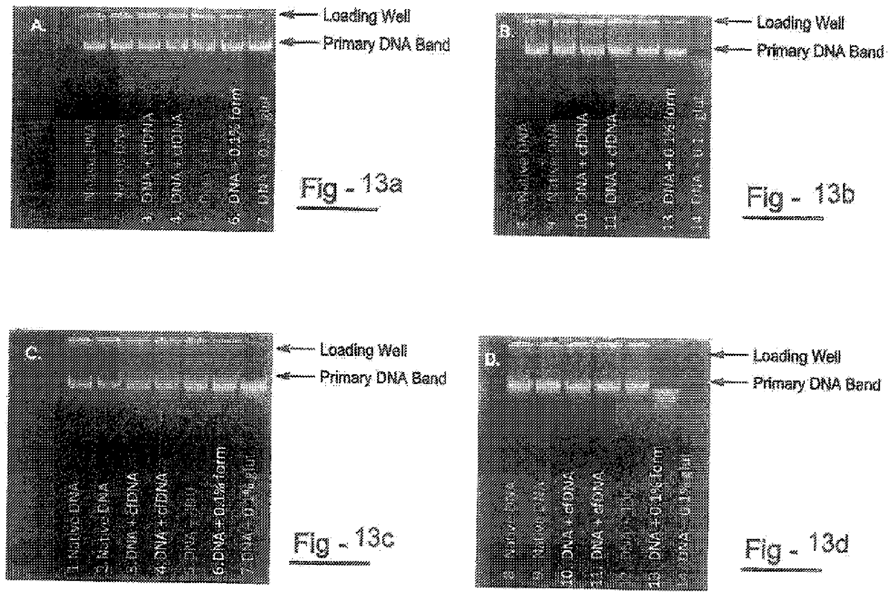

Figs. 13a-13d illustrate how a DNA formal charge is affected by formaldehyde but not with CF-DNA-BCT reagent an exemplary protective agent composition within the present teachings. There is shown results expected from gel electrophoresis of native-DNA (control), CF-DNA-BCT (an exemplary protective agent composition within the present teachings) preserved DNA, and formaldehyde preserved DNA, and held at room temperature for 7 days (Fig. 13a); at room temperature for 7 days plus heated to 60 °C for 1 hour (Fig. 13b); room temperature after 14 days (Fig. 13c); and at room temperature for 14 days plus heated to 60°C for 1 hour (Fig. 13d). Agarose gel electrophoresis of genomic DNA or fixative-treated DNA to determine changes in DNA-gel migration patterns induced by a panel of different fixatives. Fixed or untreated-DNA was incubated at room temperature for 9 (a) and 14 days (b) prior to agarose gel electrophoresis. For experiments c and d, the 7 and 14-day incubations are subjected to an additional heat treatment for 1 h at 60°C. Native, unfixed, CF-DNA reagent (an exemplary protective agent composition within the present teachings), and IDU-treated DNA (Figure 9 a-d, lanes 1-2; 8-9, lanes 3-4; 10-11, and 5; 12, respectively) are unaffected by any differences in experimental conditions. This data supports that CF-DNA BCT reagent and IDU do not change the DNA formal charge and thereby do not affect the electrophoretic migration of the primary DNA band (lower arrow, marked "Primary DNA Band") as compared to control, non-fixed, DNA. Conversely, treatment with 0.1% formaldehyde and 0.1% glutaraldehyde reduces the apparent concentration of DNA in the loading well (upper arrow, marked "Loading Well": a and c; lane 6), which was more drastic with a glutaraldehyde treatment. When the samples are exposed to heat treatment, the apparent DNA concentrations for both the loading well (upper arrow) and the primary DNA band are drastically reduced within the gel (b and d; lane 13 formaldehyde, lane 14 glutaraldehyde). In fact, formaldehyde and glutaraldehyde induce the majority or all of the DNA that is visible in the loading well, respectively. Furthermore, the DNA appeared to be at a lower molecular weight, which suggests that DNA's formal charge is altered by formaldehyde or glutaraldehyde increasing the migration rate through the gel of the more electronegative fixative-modified DNA, or DNA fragmentation induced by formaldehyde and glutaraldehyde has occurred. Alternatively, formaldehyde and glutaraldehyde can induce degradation or damage to the DNA that affect its resolution on the gel.

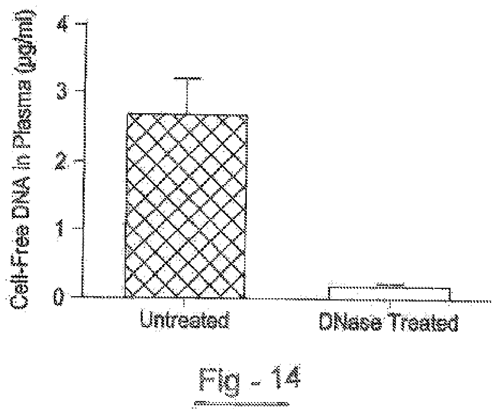

In Fig. 14, blood was drawn from 6 donors into standard K₃EDTA draw tubes. Two plasma samples from each donor were separated. One sample was treated with Proteinase K (30mAU) at 37 °C for 10 minutes and then heated at 75 °C for 15 minutes to inactivate the proteinase. DNase I (136 U) and 6 mM MgCl2 was then added to the deproteinized sample. After overnight incubation, the cfDNA concentration was measured using the Qubit dsDNA HS assay kit (Life Technologies, Grand Island, NY) and found that the fluorescence of the treated plasma was reduced by 93% as compared to the control plasma. Thus, a fluorescence based DNA assay system used to quantify cfDNA in plasma is convenient, accurate, and compatible with blood drawn into blood collection devices containing the protective agent composition described herein, which stabilizes cfDNA over 14 days of room temperature storage.

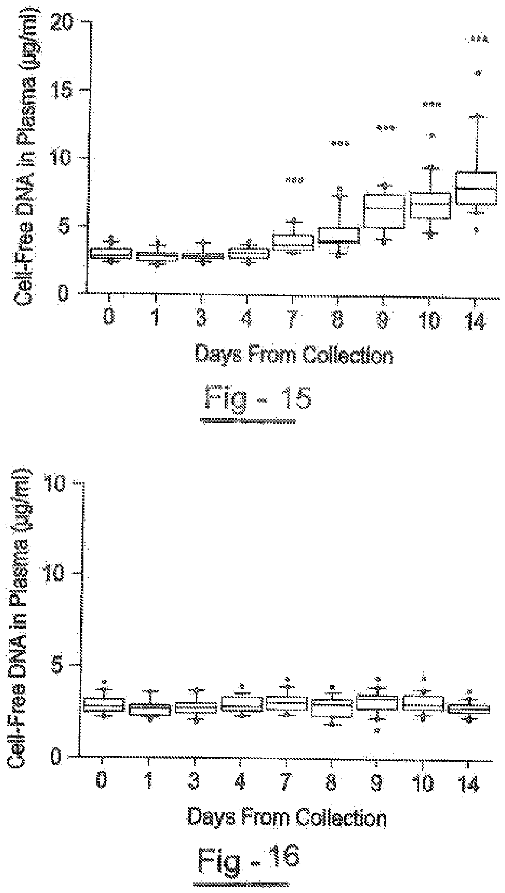

As shown in Figs. 15 and 16, blood samples from 20 donors were drawn into both K₃EDTA tubes (Fig. 15) and blood collection devices in accordance with the present teachings (Fig. 16) and stored at room temperature. At the indicated time points, an aliquot was taken from each tube and plasma separated as previously described. After plasma separation the cfDNA concentration of plasma was determined using the Qubit dsDNA HS assay. For the 14 day storage time frame, a statistically significant increase in cfDNA concentration was seen only in K₃EDTA tubes and not in the blood collection devices including the protective agent composition of the present teachings. For the box plots, the line inside of the box indicates the median value and limits of the box represent the 75th and 25th percentiles. The upper and lower error bars indicate the 10th and 90th percentiles, respectively, while the most dots indicate the any values outside the whiskers. ^{∗}*p*<0.05, ^{∗∗}*p*<0.001, ^{∗∗∗}*p*<0.0001 by paired Student's t test.

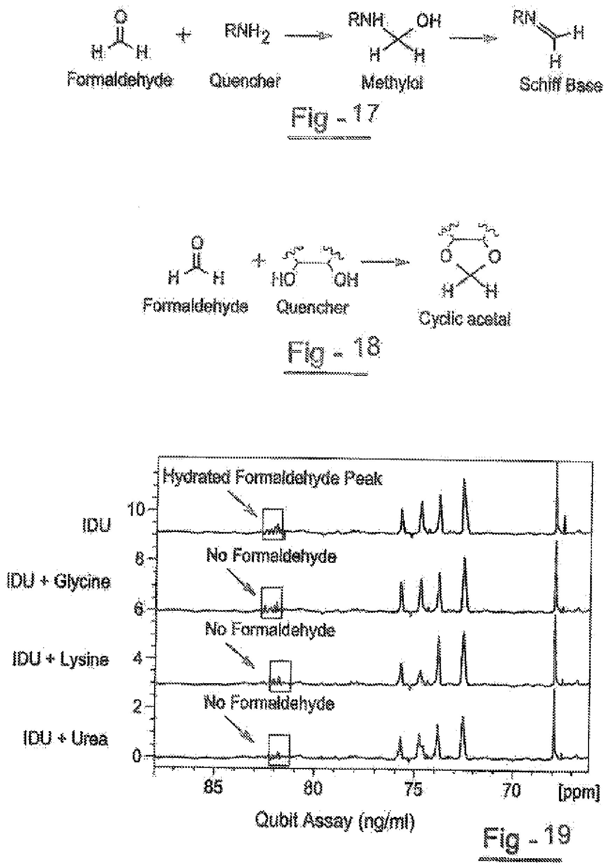

With reference to Fig. 17, formaldehyde fixation can be regulated by using formaldehyde-quenching compounds (as discussed herein) such as amines (e.g. methylamine, CH3NH2) or other amino-derivatives such as amide (e.g. urea, H2NCONH2), amino acids (e.g. glycine, HOOCCH2NH2) or ammonium salts. By reacting the quenching agent and formaldehyde as shown, products including methylol and Schiff bases are formed as depicted. In theory, any compound with electron rich functional group that can react with electron deficient carbonyl functional group of formaldehyde, can quench formaldehyde. For example, as shown in Fig. 18, cis-diol compounds can quench formaldehyde and yield cyclic acetal.

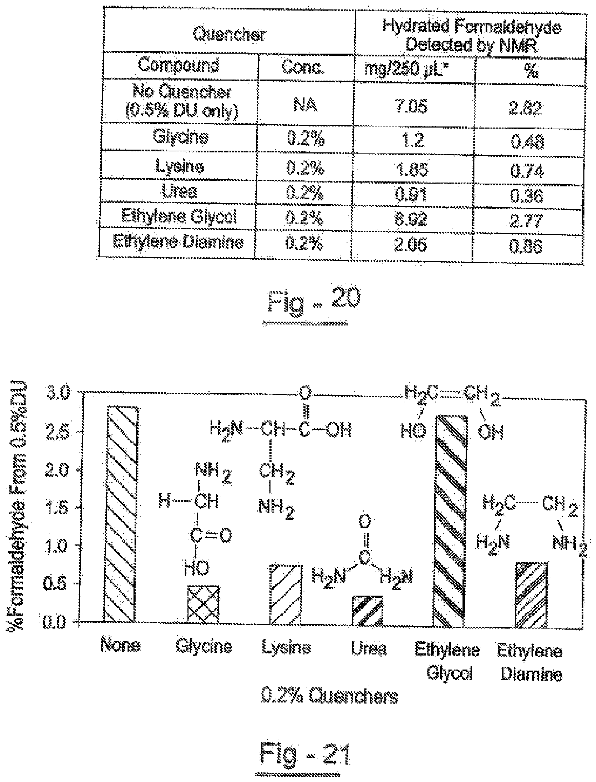

Fig. 19 shows formaldehyde released by 47.5 imidazolidinyl urea is completely quenched by glycine, lysine and urea. The ¹³C NMR spectra of imidazolidinyl urea in the presence of various quenchers is shown. The ¹³C signal of hydrated formaldehyde from imidazolidinyl urea is indicated in the shaded area at chemical shift position of 81.9 ppm. The peak is not present when glycine, lysine or urea is present in the medium. However, quenching efficiencies of the various quenching agents discussed herein are different. Diazolidinyl urea is used in Fig. 20 to determine their quenching efficiency. Fig. 20 shows relative efficiency to quench formaldehyde released from 0.5% diazolidinyl urea solution, as may be included in the protective agent composition disclosed herein.

Fig. 21 shows formaldehyde concentrations of diazolidinyl urea (0.5%) in the presence of various quenchers. The concentrations are calculated using NMR method based on peak integration.

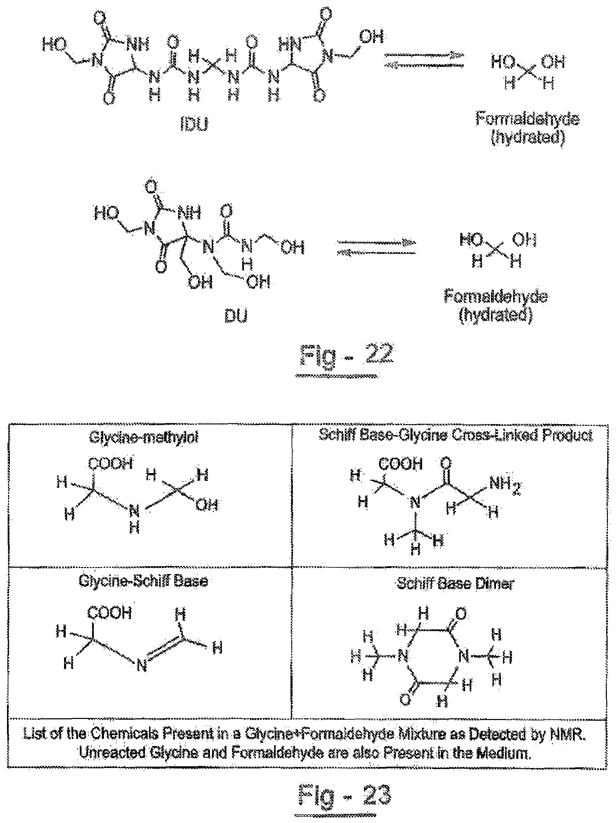

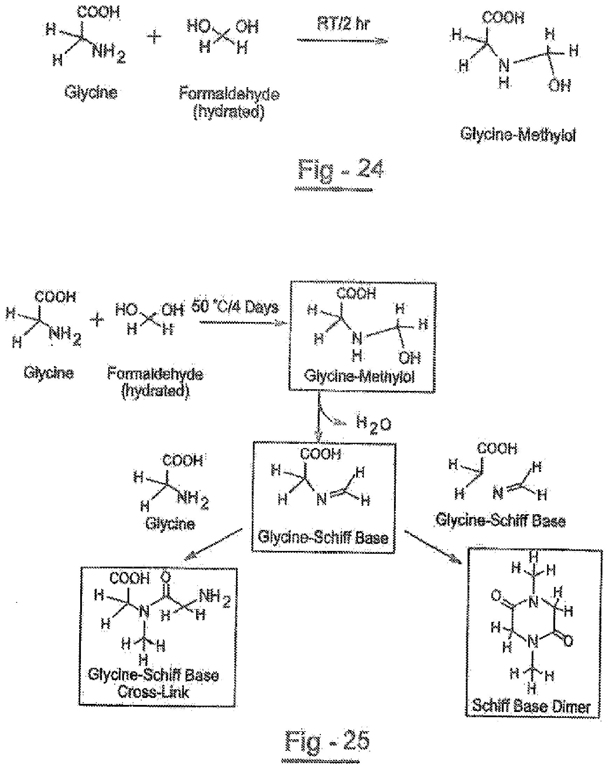

Fig. 22 depicts free hydrated formaldehyde products present in both diazolidinyl urea and imidazolidinyl urea. NMR spectroscopic study showed that when glycine (5%) and formaldehyde (5%) are mixed together, methylol is formed as shown at Fig. 23. As further shown at Fig. 23, if the glycine and formaldehyde mixture is heated at 50°C for 4 days, additional reaction products such as Schiff base, Schiff Base-glycine cross-linked product and Schiff base dimer are also formed. When glycine is added to a solution of diazolidinyl urea or imidazolidinyl urea, it reacts with the released formaldehyde from diazolidinyl urea and imidazolidinyl urea as shown for example at Fig. 24. NMR studies can detect the presence of glycine methylol and Schiff base in a mixture of imidazolidinyl urea and glycine solutions, as shown for example at Fig. 25.

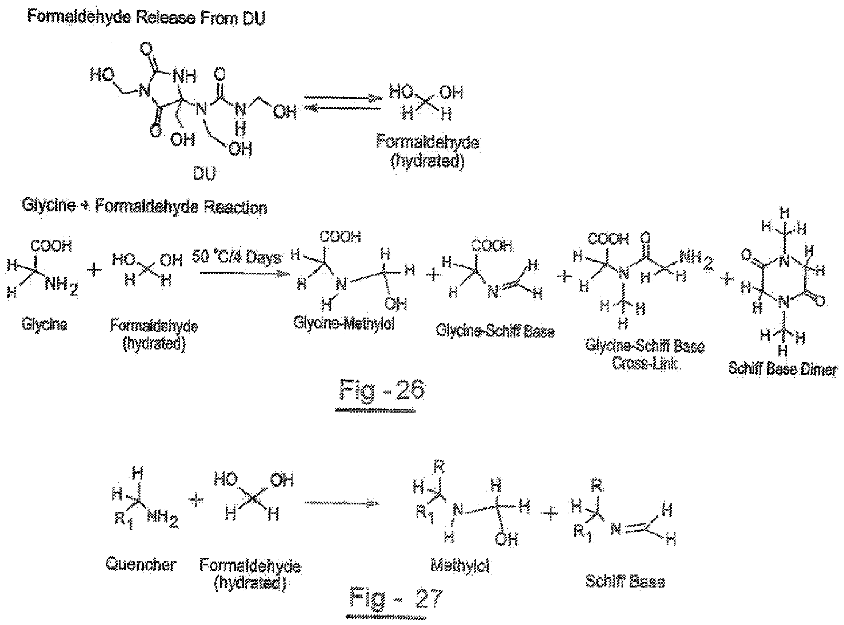

As discussed herein, diazolidinyl urea, in aqueous solution, is also known to release formaldehyde (see Fig. 26). Glycine is added to quench the released formaldehyde from diazolidinyl urea. NMR studies show that the products formed from glycine and formaldehyde are glycine-methylol, glycine-Schiff base, Glycine-Schiff base dimer, crosslinked glycine-Schiff base, as shown for Example in Fig. 26. In a mixture of diazolidinyl urea and glycine all of the above mentioned chemicals can be present in various concentrations.

In addition to glycine, several compounds can be used as formaldehyde quencher (see Fig. 27). Example of such formaldehyde quencher may include but not limited to any reactive amine group-containing compounds including urea, ammonium salts, primary and secondary amines, lysine, arginine, and/or nucleobases (i.e., adenine, guanine, cytosine, and thymine), and polyamines (i.e., spermidine). The products obtained from the reaction formaldehyde, released from diazolidinyl urea, with the above mentioned or any other amine-group containing quenchers may include corresponding methylol and Schiff base. The nature of the other bi-products may vary depending on the quencher.

As can be further appreciated from the above, the teachings herein envision methods for analyzing DNA in an aldehyde-free biological sample. The methods may include one or more steps of contacting a sample containing DNA with a protective agent composition that includes imidazolidinyl urea and one or more quenching agent as described herein. Thereafter, the sample may be treated by one or more steps for analyzing DNA.

For instance, there may be a step of contacting the sample with a fluorescing dye that selectively binds with double helix DNA, and thereafter performing an assay on the DNA that includes performing fluorescence spectroscopy, circular dichroism spectroscopy, or both to analyze the DNA of the sample. It is expected that after a period of at least about 6, 12, 24, 48, 96 hours or longer (e.g., 1 week or even two weeks later) from the time of the sample that has been contacted with the stabilizer composition it exhibits a fluorescence intensity that is within about 20% of the intensity of native DNA (at time of sample procurement) that has not been subjected to the above contacting step. It is expected that after a period of at least about 6, 12, 24, 48, 96 hours or longer (e.g., 1 week or even two weeks later) from the time of the sample that has been contacted with the stabilizer composition it exhibits an ellipticity corresponding substantially with that of native DNA (at time of sample procurement) that has not been subjected to a contacting step.

There may be a step of amplifying DNA resulting from the contacting step by PCR, wherein after a period of at least about 6, 12, 24, 48, 96 hours or longer (e.g., 1 week or even two weeks later) from the time of the sample that has been contacted with the agent exhibits an amplification efficiency that is within about 20% of that of native DNA (at time of sample procurement) that has not been subjected to a contacting step.

The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the invention. The scope of the invention should, therefore, be determined not only with reference to the above description, but should instead be determined with reference to the appended claims, along with the full scope of equivalents to which such claims are entitled. Other combinations are also possible as will be gleaned from the following claims, which are also hereby incorporated by reference into this written description. As to all of the foregoing general teachings, as used herein, unless otherwise stated, the teachings envision that any member of a genus (list) may be excluded from the genus; and/or any member of a Markush grouping may be excluded from the grouping.

Unless otherwise stated, any numerical values recited herein include all values from the lower value to the upper value in increments of one unit provided that there is a separation of at least 2 units between any lower value and any higher value. As an example, if it is stated that the amount of a component, a property, or a value of a process variable such as, for example, temperature, pressure, time and the like is, for example, from 1 to 90, preferably from 20 to 80, more preferably from 30 to 70, it is intended that intermediate range values (for example, 15 to 85, 22 to 68, 43 to 51, 30 to 32 etc.) are within the teachings of this specification. Likewise, individual intermediate values are also within the present teachings. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner. As can be seen, the teaching of amounts expressed as "parts by weight" herein also contemplates the same ranges expressed in terms of percent by weight and vice versa. Thus, an expression in the Detailed Description of the Invention of a range in terms of at "x" parts by weight of the resulting polymeric blend composition" also contemplates a teaching of ranges of same recited amount of "x" in percent by weight of the resulting polymeric blend composition."

Unless otherwise stated, all ranges include both endpoints and all numbers between the endpoints. The use of "about" or "approximately" in connection with a range applies to both ends of the range. Thus, "about 20 to 30" is intended to cover "about 20 to about 30", inclusive of at least the specified endpoints. Concentrations of ingredients identified in Tables herein may vary ±10%, or even 20% or more and remain within the teachings.

The term "consisting essentially of" to describe a combination shall include the elements, ingredients, components or steps identified, and such other elements ingredients, components or steps that do not materially affect the basic and novel characteristics of the combination. The use of the terms "comprising" or "including" to describe combinations of elements, ingredients, components or steps herein also contemplates embodiments that consist essentially of, or even consist of the elements, ingredients, components or steps. Plural elements, ingredients, components or steps can be provided by a single integrated element, ingredient, component or step. Alternatively, a single integrated element, ingredient, component or step might be divided into separate plural elements, ingredients, components or steps. The disclosure of "a" or "one" to describe an element, ingredient, component or step is not intended to foreclose additional elements, ingredients, components or steps. All references herein to elements or metals belonging to a certain Group refer to the Periodic Table of the Elements published and copyrighted by CRC Press, Inc., 1989. Any reference to the Group or Groups shall be to the Group or Groups as reflected in this Periodic Table of the Elements using the IUPAC system for numbering groups.

Even if not expressly stated, teachings from a description of one embodiment may be combined with teachings for other embodiments unless the description makes clear that such embodiments are mutually exclusive, or that the resulting combination would be clearly inoperative in the absence of unreasonable experimentation.

## Claims

1. A method of stabilizing a biological sample for analysis, comprising the steps of:
a. obtaining in a sample collection container a biological sample from a subject selected from a victim of a crime, a suspect of a crime, a subject undergoing detection and/or monitoring of a cancerous condition, a subject undergoing detection and/or monitoring of a neurogenerative condition, a subject undergoing detection and/or monitoring of a psychiatric condition, or a subject who is not pregnant, wherein the biological sample includes at least one circulating cell-free first nucleic acid from the subject;
b. contacting the biological sample while within the sample collection container with a protective agent composition that includes
- at least one preservative agent selected from diazolidinyl urea, imidazolidinyl urea, dimethoylol-5,5-dimethylhydantoin, dimethylol urea, 2-bromo-2.-nitropropane-1,3-diol, oxazolidines, sodium hydroxymethyl glycinate, 5-hydroxymethoxymethyl-1-laza-3,7-dioxabicyclo-[3.3.0]octane, 5-hydroxymethyl-1-1aza-3,7dioxabicyclo[3.3.0]octane, 5-hydroxypoly-[methyleneoxy]methyl-1-1aza-3,7dioxabicyclo [3 .3.0] octane, quaternary adamantine or any combination thereof,
- an optional anticoagulant, and
- a quenching agent to form a mixture that includes the protective agent composition and the sample, wherein the contacting step includes employing as the quenching agent an ingredient selected from glycine, lysine, ethylene diamine, arginine, urea, adenine, guanine, cytosine, thymine, spermidine, or any combination thereof;
c. quenching any free formaldehyde that may be present with the quenching agent from the protective agent composition so that the free formaldehyde reacts to form a reaction product that is inert to the first nucleic acid of the biological sample, the resulting mixture is devoid of any aldehyde, and nucleic acids within the sample are suitable for polymerase chain reaction and DNA sequencing, and DNA and are substantially devoid of aldehyde induced (i) nucleic acid (e.g., DNA) to protein cross-linking, (ii) nucleic acid (e.g., DNA) to nucleic acid (e.g., DNA) intra-molecular and/or inter-molecular cross-links; or both (i) and (ii), to thereby form a sample that is amplifiable by polymerase chain reaction (PCR), and DNA sequencing;
wherein the biological sample comes from or is in the initial form of one or more biological matter selected from urine, saliva, feces, synovial fluid, cerebrospinal fluid, or peritoneal fluid.

2. The method of claim 1, wherein the contacting step includes employing as the protective agent composition, a composition that includes:
a. imidazolidinyl urea in an amount of about 0.1 to about 1.0 % by weight of the total composition;
b. optionally, ethylenediaminetetraacetic acid in an amount of about 0.05 to about 0.75% by weight of the total composition; and
c. a quenching agent in an amount sufficient to react with any free formaldehyde that arises from the imidazolidinyl urea form a reaction product that will not react to denature any protein of the biological sample.

3. The method of any of claims 1 or 2, wherein the contacting step includes employing as the protective agent composition, a composition that includes an amount of about 10 parts by weight of the preservative agent per about 1 parts by weight of the quenching agent.

4. The method of any of claims 1 through 3, wherein in the contacting step at least one functional group of the quenching agent is capable of reacting with an electron deficient functional group of formaldehyde.

5. The method of any of claims 1 through 4, wherein the contacting step includes employing as the quenching agent an ingredient selected from glycine, lysine, ethylene diamine, urea or any combination thereof.

6. The method of any of claims 1 through 5, wherein the quenching step includes reacting any free formaldehyde for forming a methylol, imine Schiff base, a Schiff base-quencher cross-link reaction product, a Schiff base dimer, or any combination thereof.

7. The method of any of claims 1 through 6, wherein the biological sample is urine.

8. The method of any of claims 1 through 7, wherein the method includes detecting carcinomas, leukemia, and/or lymphoma.

9. The method of any of claims 1 through 8, wherein the method includes detecting abnormal methylation for breast cancer, prostate cancer, gastric cancer, ovarian, colorectal cancer, bladder cancer, testicular cancer, esophageal cancer, melanoma or other cancers.

10. The method of any of claims 1 through 9, wherein the nucleic acid is RNA or DNA.

11. The method of any of claims 1 through 10, wherein the method includes isolating RNA and detecting the absence or presence of cancer.

12. The method of any of claims 1 through 11, wherein the method includes sequencing.

## Patentansprüche

1. Verfahren zur Stabilisierung einer biologischen Probe zur Analyse, umfassend die Schritte:
a. Erhalten einer biologischen Probe in einem Probensammelbehälter von einem Subjekt, das ausgewählt wird aus einem Opfer eines Verbrechens, einem Verdächtigen eines Verbrechens, einem Subjekt, das sich dem Nachweis und/oder der Überwachung eines krebsartigen Zustands unterzieht, einem Subjekt, das sich dem Nachweis und/oder der Überwachung eines neurogenerativen Leidens unterzieht, einem Subjekt, das sich dem Nachweis und/oder der Überwachung eines psychiatrischen Leidens unterzieht, oder einem Subjekt, das nicht schwanger ist, wobei die biologische Probe mindestens eine zirkulierende zellfreie erste Nukleinsäure von dem Subjekt enthält;
b. Kontaktieren der biologischen Probe, während sie sich in dem Probensammelbehälter befindet, mit einer Schutzmittelzusammensetzung, die umfasst
- wenigstens ein Konservierungsmittel, das ausgewählt wird aus Diazolidinylharnstoff, Imidazolidinylharnstoff, Dimethylol-5,5-dimethylhydantoin, Dimethylharnstoff, 2-Brom-2-nitropropan-1,3-diol, Oxazolidinen, Natriumhydroxymethylglycinat, 5-Hydroxymethox-ymethyl-1-1aza-3,7-dioxabicyclo[3.3.0]octan, 5-Hydroxymethyl-1-1aza-3,7-dioxa-bicyclo[3.3.0]octan, 5-Hydroxypoly[methylenoxy]methyl-1-1aza-3,7dioxabicyclo[3.3.0]octan, quaternäres Adamantin, oder eine beliebige Kombination davon;
- gegebenenfalls ein Antikoagulans;
- ein Quenchmittel, um ein Gemisch zu bilden, das die Schutzmittelzusammensetzung und die Probe enthält, wobei der Kontaktschritt die Verwendung als Quenchmittel eines Bestandteils ausgewählt aus Lysin, Ethylendiamin, Arginin, Harnstoff, Adenin, Guanin, Cytosin, Thymin, Spermidin oder einer beliebigen Kombination davon umfasst;
c. Quenchen von jeglichem freien Formaldehyd, das vorliegen kann, aus der Schutzmittelzusammensetzung mit Quenchmittel, sodass das freie Formaldehyd reagiert, um ein Reaktionsprodukt zu bilden, das inert gegenüber der Nukleinsäure der biologischen Probe ist; und das entstehende Gemisch frei von jeglichem Aldehyd ist, und die Nukleinsäuren in der Probe für die Polymerase-Kettenreaktion und DNA-Sequenzierung geeignet sind, und die DNA frei von Aldehyd-induzierten (i) Nukleinsäure (zum Beispiel DNA)-Protein-Vernetzungen, (ii) intramolekularen und/oder intermolekularen Nukleinsäure (zum Beispiel DNA)-Nukleinsäure (zum Beispiel DNA)-Vernetzungen, oder sowohl (i) als auch (ii) ist, um dadurch eine Probe zu bilden, die durch Polymerasekettenreaktion (PCR) und DNA-Sequenzierung amplifizierbar ist;
wobei die biologische Probe von einem oder mehreren biologischen Stoffen stammt oder in der ursprünglichen Form in einem oder mehreren biologischen Stoffen vorliegt, die aus Urin, Speichel, Fäkalien, Synovialflüssigkeit, Cerebrospinalflüssigkeit oder Peritonealflüssigkeit ausgewählt wird.

2. Das Verfahren gemäß Anspruch 1, wobei der Kontaktschritt die Verwendung als Schutzmittelzusammensetzung einer Zusammensetzung umfasst, die umfasst:
a. Imidazolidinylharnstoff als Konservierungsmittel in einer Menge von ungefähr 0,1 bis ungefähr 1,0 Gew.-%, bezogen auf die Gesamtzusammensetzung;
b. gegebenenfalls Ethylendiamintetraessigsäure als Antikoagulans in einer Menge von ungefähr 0,05 bis ungefähr 0,75 Gew.-%, bezogen auf die Gesamtzusammensetzung; und
c. ein Quenchmittel in einer Menge, die ausreicht, um mit jeglichem freien Formaldehyd, das aus dem Imidazolidinylharnstoff entsteht, zu reagieren und ein Reaktionsprodukt zu bilden, das mit keinem Protein der biologischen Probe eine Denaturierungsreaktion eingeht.

3. Das Verfahren gemäß Anspruch 1 oder 2, wobei der Kontaktschritt die Verwendung einer Schutzmittelzusammensetzung umfasst, wobei die Zusammensetzung eine Menge von ungefähr 10 Gewichtsteilen des Konservierungsmittels pro ungefähr 1 Gewichtsteil des Quenchmittels enthält.

4. Das Verfahren gemäß einem oder mehreren der Ansprüche 1 bis 3, wobei im Kontaktschritt mindestens eine funktionelle Gruppe des Quenchmittels mit einer elektronenarmen funktionellen Gruppe des Formaldehyds reagiert.

5. Das Verfahren gemäß einem oder mehreren der Ansprüche 1 bis 4, wobei der Kontaktschritt die Verwendung als Quenchmittel eines Bestandteils ausgewählt aus Lysin, Ethylendiamin, Arginin, Harnstoff, Adenin, Guanin, Cytosin, Thymin, Spermidin oder eine beliebige Kombination davon umfasst

6. Das Verfahren gemäß einem oder mehreren der Ansprüche 1 bis 5, wobei der Kontaktschritt das Umsetzen von jeglichem freien Formaldehyd zur Bildung eines Methylols, einer Imin-Schiffschen Base, eines Schiffschen Basen-Quenchmittel-Vernetzungsreaktionsprodukts, eines Schiffschen Base-Dimers oder einer Kombination davon umfasst.

7. Das Verfahren gemäß einem oder mehreren der Ansprüche 1 bis 6, wobei die biologische Probe Urin ist.

8. Das Verfahren gemäß einem oder mehreren der Ansprüche 1 bis 7, wobei das Verfahren den Nachweis von Karzinomen, Leukämie und/oder Lymphomen umfasst.

9. Das Verfahren gemäß einem oder mehreren der Ansprüche 1 bis 8, wobei das Verfahren den Nachweis anormaler Methylierung für Brustkrebs, Prostatakrebs, Magenkrebs, Eierstockkrebs, kolorektalem Krebs, Blasenkrebs, Hodenkrebs, Speiseröhrenkrebs, Melanom, oder anderer Krebsarten umfasst.

10. Das Verfahren gemäß einem oder mehreren der Ansprüche 1 bis 9, wobei die Nukleinsäure RNA oder DNA ist.

11. Das Verfahren gemäß einem oder mehreren der Ansprüche 1 bis 10, wobei das Verfahren die Isolierung von RNA und den Nachweis der Abwesenheit oder Anwesenheit von Krebs umfasst.

12. Das Verfahren gemäß einem oder mehreren der Ansprüche 1 bis 11, wobei das Verfahren die Sequenzierung umfasst.

## Revendications

1. Procédé de stabilisation d'un échantillon biologique pour analyse, comprenant les étapes de :
a. l'obtention, dans un récipient de prélèvement d'échantillon, d'un échantillon biologique d'un sujet choisi parmi une victime d'un crime, un suspect d'un crime, un sujet faisant l'objet d'un dépistage et/ou d'un suivi d'une affection cancéreuse, un sujet faisant l'objet d'un dépistage et/ou d'un suivi d'une affection neurogénérative, un sujet faisant l'objet d'un dépistage et/ou d'un suivi d'une affection psychiatrique, ou un sujet qui n'est pas enceinte, dans lequel l'échantillon biologique comporte au moins un premier acide nucléique acellulaire circulant du sujet ;
b. la mise en contact de l'échantillon biologique, lorsqu'il est dans le récipient de prélèvement d'échantillon, avec une composition d'agent de protection qui comporte
- au moins un agent de conservation choisi parmi la diazolidinylurée, l'imidazolidinylurée, la diméthoylol-5,5-diméthyl-hydantoïne, la diméthylolurée, le 2-bromo-2-nitropropane-1,3-diol, des oxazolidines, l'hydroxyméthylglycinate de sodium, le 5-hydroxyméthoxyméthyl-1-laza-3,7-dioxabicyclo-[3.3.0]octane, le 5-hydroxyméthyl-1-laza-3,7-dioxabicyclo[3.3.0]octane, le 5-hydroxypoly-[méthylèneoxy]méthyl-1-laza-3,7-dioxabicyclo[3.3.0]octane, l'adamantine quaternaire ou toute combinaison de ceux-ci,
- un anticoagulant facultatif, et
- un agent de désactivation pour former un mélange qui comporte la composition d'agent de protection et l'échantillon, dans lequel l'étape de mise en contact comporte l'utilisation, comme étant l'agent de désactivation, d'un ingrédient choisi parmi la glycine, la lysine, l'éthylène diamine, l'arginine, l'urée, l'adénine, la guanine, la cytosine, la thymine, la spermidine, ou toute combinaison de celles-ci ;
c. la désactivation de tout formaldéhyde libre qui peut être présent avec l'agent de désactivation de la composition d'agent de protection de sorte que le formaldéhyde libre réagisse pour former un produit de réaction inerte par rapport au premier acide nucléique de l'échantillon biologique, le mélange résultant est dépourvu de tout aldéhyde, et les acides nucléiques dans l'échantillon sont appropriés pour une réaction en chaîne par polymérase et au séquençage d'ADN, et l'ADN et sont sensiblement dépourvus (i) d'une réticulation de l'acide nucléique (par exemple, ADN)à la protéine induite par un aldéhyde, (ii) de réticulations intramoléculaire et/ou intermoléculaire de l'acide nucléique (par exemple, ADN) à l'acide nucléique (par exemple, ADN) ; ou à la fois de (i) et (ii), pour ainsi former un échantillon qui est amplifiable par une réaction en chaîne par polymérase (PCR) et un séquençage d'ADN ;
dans lequel l'échantillon biologique provient de ou est sous la forme initiale d'une ou plusieurs matières biologiques choisies parmi l'urine, la salive, les matières fécales, le liquide synovial, le liquide céphalo-rachidien ou le liquide péritonéal.

2. Procédé selon la revendication 1, dans lequel l'étape de mise en contact comporte l'utilisation, comme étant la composition d'agent de protection, d'une composition qui comporte :
a. l'imidazolidinylurée en une quantité d'environ 0,1 à environ 1,0 % en poids de la composition totale ;
b. éventuellement, l'acide éthylènediaminetétraacétique en une quantité d'environ 0,05 à environ 0,75 % en poids de la composition totale ; et
c. un agent de désactivation en une quantité suffisante pour réagir avec tout formaldéhyde libre qui provient de l'imidazolidinylurée forme un produit de réaction qui ne réagira pas pour dénaturer une quelconque protéine de l'échantillon biologique.

3. Procédé selon l'une quelconque des revendications 1 ou 2, dans lequel l'étape de mise en contact comporte l'utilisation, comme étant la composition d'agent de protection, d'une composition qui comporte une quantité d'environ 10 parties en poids de l'agent de conservation pour environ 1 partie en poids d'agent de désactivation.

4. Procédé selon l'une quelconque des revendications 1 à 3, dans lequel, dans l'étape de mise en contact, au moins un groupe fonctionnel de l'agent de désactivation est capable de réagir avec un groupe fonctionnel déficient en électrons du formaldéhyde.

5. Procédé selon l'une quelconque des revendications 1 à 4, dans lequel l'étape de mise en contact comporte l'utilisation, comme étant l'agent de désactivation, d'un ingrédient choisi parmi la glycine, la lysine, l'éthylène diamine, l'urée ou toute combinaison de ceux-ci.

6. Procédé selon l'une quelconque des revendications 1 à 5, dans lequel l'étape de désactivation comporte la réaction de tout formaldéhyde libre pour former un méthylol, une base de Schiff (imine), un produit de réaction de réticulation de base de Schiff-agent de désactivation, un dimère de base de Schiff ou toute combinaison de ceux-ci.

7. Procédé selon l'une quelconque des revendications 1 à 6, dans lequel l'échantillon biologique est l'urine.

8. Procédé selon l'une quelconque des revendications 1 à 7, dans lequel le procédé comporte la détection de carcinomes, de leucémie et/ou de lymphome.

9. Procédé selon l'une quelconque des revendications 1 à 8, dans lequel le procédé comporte la détection d'une méthylation anormale pour le cancer du sein, le cancer de la prostate, le cancer gastrique, le cancer de l'ovaire, le cancer colorectal, le cancer de la vessie, le cancer des testicules, le cancer de l'œsophage, le mélanome ou d'autres cancers.

10. Procédé selon l'une quelconque des revendications 1 à 9, dans lequel l'acide nucléique est un ARN ou un ADN.

11. Procédé selon l'une quelconque des revendications 1 à 10, dans lequel le procédé comporte l'isolement d'un ARN et la détection de l'absence ou de la présence d'un cancer.

12. Procédé selon l'une quelconque des revendications 1 à 11, dans lequel le procédé comporte un séquençage.
